# DEMANDE DE BREVET EUROPEEN

(11) **EP 1 070 716 A1**
(43) Date de publication de la demande: **24.01.2001**
(21) Numéro de dépôt: 00402073.1
(22) Date de dépôt: 21.07.2000
(51) Int. Cl.: C07D 471/04, A61K 31/437, A61P 25/00

(54) **Nouveaux dérivés de beta-carboline, leur procédé de préparation et les compositions pharmaceutiques qui les contiennent**

(30) Priorité: 23.07.1999 FR 9909576
(71) Demandeur: ADIR ET COMPAGNIE, 92415 Courbevoie Cédex (FR)
(72) Inventeur: Goldstein, Solo, 92150 Suresnes (FR); Poissonnet, Guillaume, 91400 Orsay (FR); Parmentier, Jean-Gilles, 92130 Issy les Moulineaux (FR); Brion, Jean-Daniel, 95320 Saint leu la Foret (FR); Millan, Mark, 78230 Le Pecq (FR); Dekeyne, Anne, 78470 Saint Remy les Chevreuses (FR); Boutin, Jean, 92150 Suresnes (FR)

(57) **Abrégé**

Composés de formule (I): 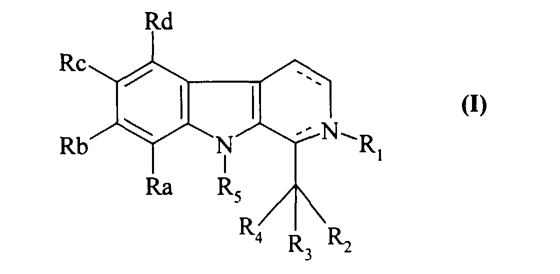 dans laquelle :
----représente une liaison simple ou double,
R₁ représente un atome d'hydrogène, un groupement alkyle, -R₆-aryle, -R₆-cycloalkyle, -R₆-hétérocycle, -CO₂R₇-, -COR₈ ou -CONHR₈ dans lesquels R₆, R₇ et R₈ sont tels que définis dans la description,
R₂ représente un groupement cyano, mono ou dialkylaminoalkylaminocarbonyle, -CO₂R₈, -CONHR₈, -NR₈R₉, -NHCO₂R₇, ou -COR₈ dans lesquels R₇, R₈ et R₉ sont tels que définis dans la description,
R₃ et R₄ forment ensemble un cycloalkyle (C₃-C₁₀),
R₅ représente un atome d'hydrogène, un groupement alkyle ou arylalkyle,
Ra, Rb, Rc, Rd, identiques ou différents, représentent un groupement tel que défini dans la description,
leurs isomères ainsi que leurs sels d'addition à un acide ou à une base pharmaceutiquement acceptable.

Médicaments.

## Description

La présente invention concerne de nouveaux dérivés de β-carboline, leur procédé de préparation et les compositions pharmaceutiques qui les contiennent. Ces nouveaux composés possèdent une activité sérotoninergique sur les récepteurs de la famille 5-HT₂.

La sérotonine est un neurotransmetteur qui agit sur les récepteurs 5-HT (5-hydroxytryptamine) à la fois au niveau central et périphérique. A ce jour, quatorze sous-types de récepteur de la sérotonine ont été identifiés et classés au sein de sept familles, 5-HT₁ à 5-HT₇. Parmi la famille des 5-HT₂, les sous-types 5-HT_{2A}, 5-HT_{2B} et 5-HT_{2C} sont connus. Ces sous-types jouent un rôle similaire dans leur spécificité pour un grand nombre de ligands (Trends. Pharmacol. Sci., 1995, 16, 105-110, Neuropharmacology, 1994, 33, 275-317).

Les composés pouvant moduler l'activité des récepteurs 5-HT₂ et notamment des récepteurs 5-HT_{2C} et 5-HT_{2B} sont ainsi susceptibles d'être utilisés dans le traitement des maladies telles que les troubles du sommeil (Psychopharmacology, 1989, 97, 436-442 ; Neuropharmacol., 1994, 33, 467-471), les troubles de l'appétit (Psychopharmacology, 1997, 133, 309-312), les attaques de paniques, les phobies, l'anxiété (Br. J. Pharmacol., 1996, 117, 427-434 ; Neuropharmacology, 1997, 36, 793-802), la dépression (Biol. Psychiatry, 1996, 39, 1000-1008 ; Neuroscience, 1999, 91(2), 587-597), les troubles impulsifs et agressifs (Pharm. Biochem. Behavior, 1991, 39, 729-736), les troubles sexuels (Clinical Neuropharmacology, 1997 20(3), 210-214), la migraine (Progress in Drug Research, 1998, 51, 219-244, ed. Springer Verlay), la schizophrénie et la psychose (Eur. J. Pharm., 1993, 245, 179-182 ; Biol. Psychiatry, 1998, 44, 1099-1117).

De nombreux dérivés de β-carboline ont été décrits dans la littérature. C'est le cas plus particulièrement de la demande de brevet EP 0 620 223 qui revendique des dérivés de tétrahydro-pyrido-indole, ceux-ci présentant une forte affinité pour les récepteurs 5-HT_{2C}. Les demandes de brevet EP 0 320 079 et EP 0 300 541 revendiquent quand à elles des composés β-carbolines, dihydro-β-carboline et tétrahydro-β-carboline, qui possèdent une forte activité fibrinolytique. Enfin, la demande de brevet WO 95/24200 décrit des composés possédant notamment un motif structural tétrahydro-β-carboline. Ces produits sont des antagonistes spécifiques des récepteurs 5-HT_{2B}.

Les composés de la présente invention, outre le fait qu'ils soient nouveaux, se sont révélés être de puissants ligands sélectifs des récepteurs 5-HT₂ et notamment des antagonistes 5-HT_{2C} et 5HT_{2B}, ce qui les rend donc potentiellement utiles pour le traitement de la dépression, de la psychose, de la schizophrénie, de la phobie, de l'anxiété, des attaques de panique, des troubles du sommeil, des troubles de l'appétit, des troubles impulsifs et agressifs, des troubles sexuels, et de la migraine.

Plus spécifiquement, la présente invention concerne les composés de formule (**I**): 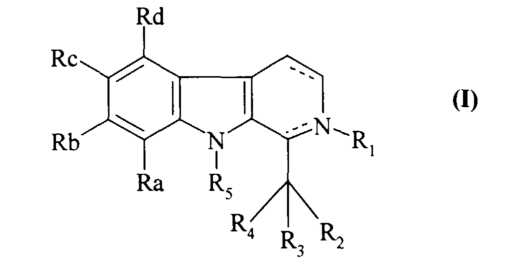 dans laquelle :
---- représente une liaison simple ou double pouvant éventuellement conférée un caractère aromatique au cycle qui les porte,
R₁ représente l'un des groupements choisis parmi :
   ◆ hydrogène,
   ◆ alkyle (C₁-C₆) linéaire ou ramifié,
   ◆ -R₆-aryle, -R₆-cycloalkyle, -R₆-hétérocycle, groupements dans lesquels R₆ représente un groupement alkylène (C₁-C₆) linéaire ou ramifié,
   ◆ -CO₂R₇ dans lequel R₇ représente un groupement alkyle (C₁-C₆) linéaire ou ramifié, aryle, cycloalkyle, un hétérocycle, un groupement -R₆-aryle, -R₆-cycloalkyle ou -R₆-hétérocycle avec R₆ tel que défini précédemment,
   ◆ -COR₈ dans lequel R₈ représente un atome d'hydrogène, un groupement alkyle (C₁-C₆) linéaire ou ramifié, aryle, cycloalkyle, un hétérocycle, un groupement -R₆-aryle, -R₆-cycloalkyle ou -R₆-hétérocycle avec R₆ tel que défini précédemment,
   ◆ et -CONH-R₈ dans lequel R₈ est tel que défini précédemment,
   ou R₁ n'existe pas lorsque l'atome d'azote qui le porte, porte déjà une double liaison intracyclique,
R₂ représente l'un des groupements choisis parmi :
   ◆ cyano,
   ◆ -CO₂R₈ dans lequel R₈ est tel que défini précédemment,
   ◆ -CONHR₈ dans lequel R₈ est tel que défini précédemment,
   ◆ monoalkyl(C₁-C₆)aminoalkyl(C₁-C₆)aminocarbonyle, dialkylamino(C₁-C₆)aminoalkyl-(C₁-C₆)aminocarbonyle, les parties alkyles de chacun de ces groupements pouvant être linéaires ou ramifiées,
   ◆ -NR₈R₉ dans lequel R₈ est tel que défini précédemment et R₉ représente un groupement tel que R₈ est défini,
   ◆ -NH-CO₂R₇ dans lequel R₇ est tel que défini précédemment,
   ◆ et -COR₈ dans lequel R₈ est tel que défini précédemment,
R₃ et R₄ forment ensemble un cycloalkyle (C₃-C₁₀),
R₅ représente un atome d'hydrogène, un groupement alkyle (C₁-C₆) linéaire ou ramifié, ou arylalkyle (C₁-C₆) linéaire ou ramifié,
Ra, Rb, Rc, Rd, identiques ou différents, indépendamment l'un de l'autre, représentent un groupement choisi parmi atome d'hydrogène, d'halogène, groupement alkyle (C₁-C₆) linéaire ou ramifié, hydroxy, alkoxy (C₁-C₆) linéaire ou ramifié, trihalogénoalkyle (C₁-C₆) linéaire ou ramifié, trihalogénoalkoxy (C₁-C₆) linéaire ou ramifié, nitro, cyano, amino, alkyle(C₁-C₆)amino linéaire ou ramifié, dialkyle(C₁-C₆)amino linéaire ou ramifié, aryle, arylalkyle (C₁-C₆) linéaire ou ramifié, carboxy, alkylcarbonyloxy (C₁-C₆) linéaire ou ramifié, acyle (C₁-C₆) linéaire ou ramifié, aryloxy, et arylalkoxy (C₁-C6) linéaire ou ramifié,
leurs isomères ainsi que leurs sels d'addition à un acide ou à une base pharmaceutiquement acceptable.

Etant entendu que :
- par cycloalkyle, on comprend un groupement mono ou bicyclique, saturé (ou comportant éventuellement une ou plusieurs insaturations, celles-ci ne conférant pas un caractère aromatique au système cyclique), comportant de 3 à 10 atomes de carbone, éventuellement substitué par un ou plusieurs groupements, identiques ou différents, choisis parmi halogène, hydroxy, alkyle (C₁-C₆) linéaire ou ramifié, et alkoxy (C₁-C₆) linéaire ou ramifié,
- par aryle, on comprend un groupement phényle, naphtyle, tétrahydronaphtyle, dihydronaphtyle, indényle ou indanyle, chacun de ces groupements étant éventuellement substitué de façon identique ou différente, par un ou plusieurs groupements choisis parmi halogène, groupements hydroxy, cyano, nitro, alkyle (C₁-C₆) linéaire ou ramifié, alkoxy (C₁-C₆) linéaire ou ramifié, amino, alkyle (C₁-C₆)amino linéaire ou ramifié, dialkyle(C₁-C₆)amino linéaire ou ramifié, aryloxy, arylalkoxy (C₁-C₆) linéaire ou ramifié, trihalogénoalkyle (C₁-C₆) linéaire ou ramifié, acyle (C₁-C₆) linéaire ou ramifié, alkoxycarbonyle (C₁-C₆) linéaire ou ramifié, alkylaminocarbonyle (C₁-C₆) linéaire ou ramifié, et oxo,
- par hétérocycle, on comprend un groupement mono ou bicyclique, saturé ou insaturé, à caractère aromatique ou non aromatique, de 5 à 12 chaînons, contenant un, deux ou trois hétéroatomes, identiques ou différents, choisis parmi oxygène, azote et soufre, étant entendu que l'hétérocycle peut être éventuellement substitué, de façon identique ou différente, par un ou plusieurs groupements choisis parmi halogène, hydroxy, alkyle (C₁-C₆) linéaire ou ramifié, alkoxy (C₁-C₆) linéaire ou ramifié, nitro, oxo, et amino (substitué éventuellement par un ou deux groupements alkyle (C₁-C₆) linéaire ou ramifié).

Parmi les hétérocycles, on peut citer à titre indicatif, et non limitatif, le groupement pyridinyle, thiényle, furyle, imidazolyle, 4H-pyranyl-4-one, pyrazinyle, pyrimidinyle, isoxazolyle, tétrazolyle, pyrrolyle, pyrazolyle, quinolyle, isoquinolyle, quinazolinyle, pyrrolidinyle, pipéridyle, pipérazinyle, 1,2,3-thiadiazolyle, ...

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif, l'hydroxyde de sodium, l'hydroxyde de potassium, la triéthylamine, la tertbutylamine, etc...

Selon une variante avantageuse, les composés préférés de l'invention sont ceux pour lesquels R₃ et R₄ forment ensemble un cycloalkyle (C₃-C₁₀), monocyclique et saturé, éventuellement substitué par un ou plusieurs groupements tels que définis précédemment. D'une façon particulièrement avantageuse, R₃ et R₄ forment ensemble un cycloalkyle (C₄-C₆) monocyclique, saturé et non substitué. Plus préférentiellement encore, R₃ et R₄ forment ensemble un cyclobutyle.

Les substituants R₁ préférés selon l'invention sont l'atome d'hydrogène, ou le groupement -COR₈ dans lequel R₈ est tel que défini dans la formule (I). Selon une variante avantageuse, le substituant R₁ préféré est le groupement -COR₈ₐ dans lequel R₈ₐ représente un groupement aryle ou un hétérocycle.

Le substituant R₂ préféré selon l'invention est le groupement -CO₂R₈ dans lequel R₈ est tel que défini dans la formule (I). Selon une variante avantageuse, le substituant R₂ préféré est le groupement -CO₂R_{8b} dans lequel R_{8b} représente un groupement alkyle (C₁-C₆) linéaire ou ramifié ou un cycloalkyle. D'une façon particulièrement avantageuse, R_{8b} représente un groupement éthyle ou un cyclopentyle.

Le substituant R₅ préféré selon l'invention est l'atome d'hydrogène.

Selon une variante particulièrement avantageuse, les composés préférés de l'invention sont les dérivés de 2,3,4,9-tétrahydro-1*H*-β-carboline de formule (**I'**): 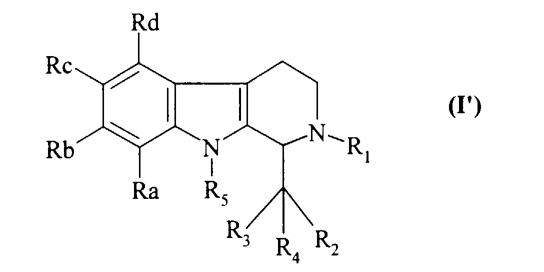 dans laquelle :
R₁, R₂, R₃, R₄, R₅, Ra, Rb, Rc et Rd sont tels que définis dans la formule (I).

Les composés préférés selon l'invention sont :
- le 1-(6-chloro-2,3,4,9-tétrahydro-1*H*-β-carbolin-1-yl)cyclobutanecarboxylate de cyclopentyle,
- le 1-(6-bromo-2,3,4,9-tétrahydro-1*H*-β-carbolin-1-yl)cyclobutanecarboxylate d'éthyle,
- le 1-[6-chloro-2-(1*H*-imidazol-5-ylcarbonyl)-2,3,4,9-tétrahydro-1*H*-β-carbolin-1-yl]cyclobutanecarboxylate d'éthyle,
- le 1-(6-méthyl-2,3,4,9-tétrahydro-1*H*-β-carbolin-1-yl)cyclobutanecarboxylate d'éthyle,
- le 1-(5,6-dichloro-2,3,4,9-tétrahydro-1*H*-β-carbolin-1-yl)cyclobutanecarboxylate d'éthyle,
- le 1-(6-chloro-2,3,4,9-tétrahydro-1*H*-β-carbolin-1-yl)cyclobutanecarboxylate d'éthyle,
- le 1-(6,7-dichloro-2,3,4,9-tétrahydro-1*H*-β-carbolin-1-yl)cyclobutanecarboxylate d'éthyle,
- et le 1-(6-méthoxy-2,3,4,9-tétrahydro-1*H*-β-carbolin-1-yl)cyclobutanecarboxylate d'éthyle.

Les isomères ainsi que les sels d'addition, à un acide ou à une base pharmaceutiquement acceptable, des composés préférés font partie intégrante de l'invention.

L'invention s'étend également au procédé de préparation des composés de formule (I) caractérisé en ce qu'on utilise comme produit de départ un composé de formule (**II**) : 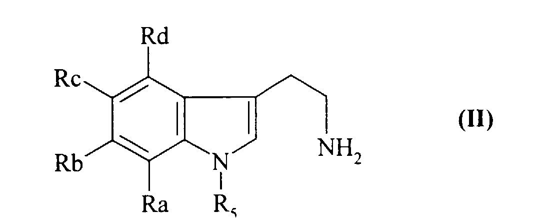 dans lequel Ra, Rb, Rc, Rd et R₅ ont la même signification que dans la formule (I),
composé de formule (II) qui est mis à réagir, selon des conditions de synthèse de type couplage peptidique, avec un composé de formule (**III**) : 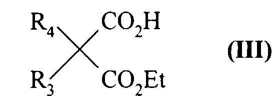 dans laquelle R₃ et R₄ sont tels que définis dans la formule (I),
pour conduire au composé de formule (**IV**) : 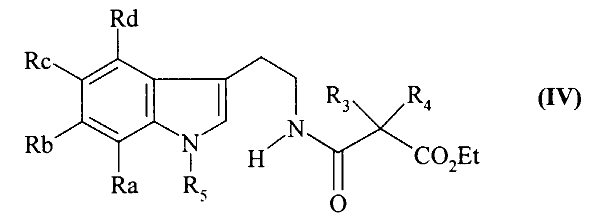 dans laquelle Ra, Rb, Rc, Rd, R₃, R₄ et R₅ sont tels que définis précédemment, composés de formule (IV) que l'on traite en présence d'oxychlorure de phosphore dans un solvant tel que le toluène ou le benzène, pour conduire aux composés de formule (**I/a**), cas particulier des composés de formule (I): 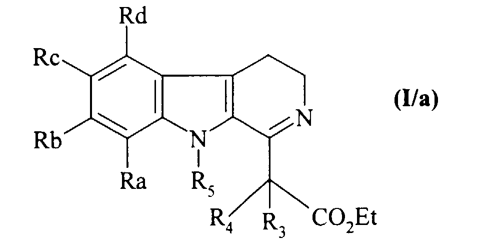 dans laquelle Ra, Rb, Rc, Rd, R₃, R₄ et R₅ sont tels que définis précédemment, composés de formule (I/a) qui sont :
* soit réduits selon des conditions classiques de synthèse organique, pour conduire aux composés de formule (**I/b**), cas particulier des composés de formule (I): 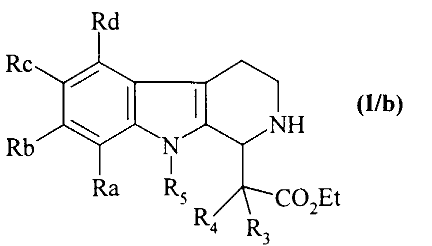 dans laquelle Ra, Rb, Rc, Rd, R₃, R₄ et R₅ sont tels que définis précédemment,
   composés de formule (I/b) que l'on traite en condition basique en présence d'un composé de formule (**V**) :

   R₁ - X **(V)**

   dans laquelle R₁ est tel que défini dans la formule (I) et X représente un groupe partant usuellement utilisé en synthèse organique,
   pour conduire aux composés de formule (**I/c**), cas particulier des composés de formule (I) : 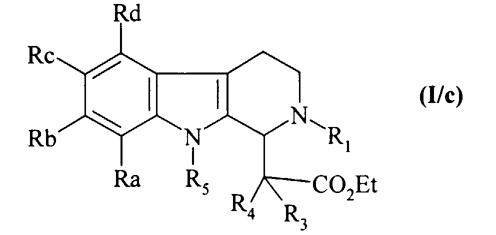
* soit soumis à des agents oxydants usuellement utilisés en synthèse organique pour conduire aux composés de formule (**I/d**), cas particulier des composés de formule (I): 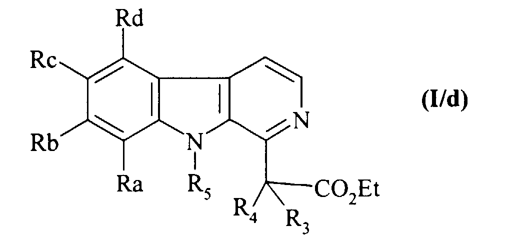 dans laquelle Ra, Rb, Rc, Rd, R₃, R₄ et R₅ sont tels que définis précédemment,
   l'ensemble des composés de formule (I/a), (I/b), (I/c) et (I/d) forment les composés de formule (**I/e**), cas particulier des composés de formule (I): 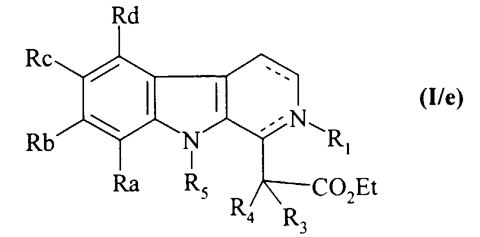 dans laquelle Ra, Rb, Rc, Rd, R₁, R₃, R₄ et R₅ ont la même définition que dans la formule (I), composé de formule (I/e),
* que l'on soumet à des conditions de transestérification en présence d'un acide de Lewis, et d'un composé de formule (**VI**) :

   R₇ - OH **(VI)**

   dans laquelle R₇ est tel que défini dans la formule (I),
   pour conduire, aux composés de formule (**I/f**), cas particulier des composés de formule (I): 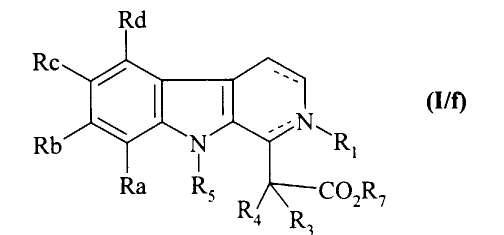 dans laquelle Ra, Rb, Rc, Rd, R₁, R₃, R₄, R₅ et R₇ sont tels que définis précédemment,
* ou que l'on hydrolyse en condition basique, pour conduire aux composés de formule (**I/g**), cas particulier des composés de formule (I): 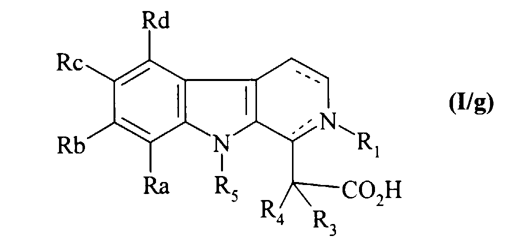 dans laquelle Ra, Rb, Rc, Rd, R₁, R₃, R₄ et R₅ sont tels que définis précédemment, composé de formule (I/g) que l'on traite :
   ◆ *soit,* selon des conditions classiques d'amidification, par un composé de formule (**VII**):

      R₈ - NH₂ **(VII)**

      dans laquelle R₈ est tel que défini dans la formule (I), pour conduire aux composés de formule (**I/h**), cas particulier des composés de formule (I): 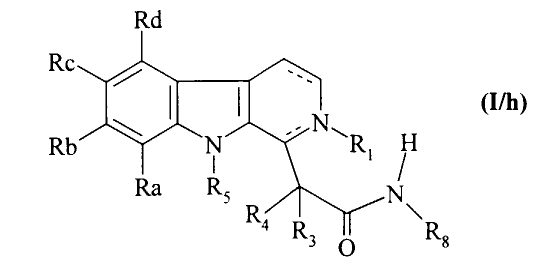 dans laquelle Ra, Rb, Rc, Rd, R₁, R₃, R₄, R₅ et R₈ sont tels que définis précédemment, composés de formule (I/h), dans le cas particulier où R₈ représente un atome d'hydrogène, dont la fonction amide primaire est transformée en fonction nitrile selon des conditions classiques de la synthèse organique, pour conduire aux composés de formule (**I/i**), cas particulier des composés de formule (I): 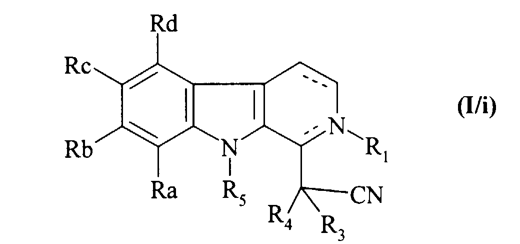 dans laquelle Ra, Rb, Rc, Rd, R₁, R₃, R₄ et R₅ sont tels que définis précédemment,
   ◆ *soit* dont on transforme la fonction acide carboxylique en aldéhyde, par une suite de réaction de réduction puis oxydation selon des conditions usuelles de la chimie organique, pour conduire aux composés de formule (**I/j**), cas particulier des composés de formule (I): 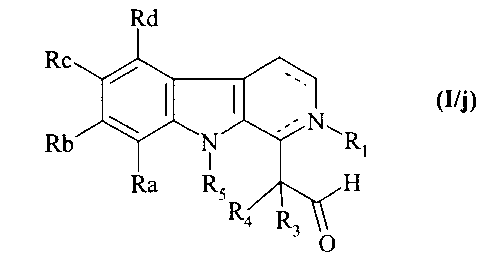 dans laquelle Ra, Rb, Rc, Rd, R₁, R₃, R₄ et R₅ sont tels que définis précédemment, composés de formule (I/j) qui est mis en présence d'un composé de formule (**VIII**):

      R₇ - M - X **(VIII)**

      dans laquelle R₇ est tel que défini dans la formule (I), M représente un atome de métal tel qu'un métal alcalin ou du magnésium et X représente un groupement partant tel qu'un atome d'halogène, pour conduire intermédiairement aux composés de formule (**IX**): 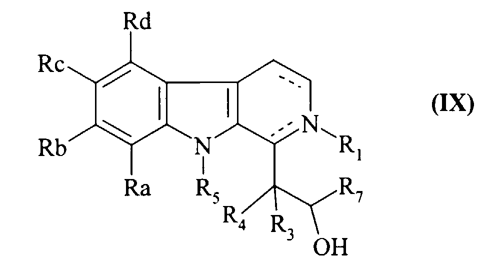 dans laquelle Ra, Rb, Rc, Rd, R₁, R₃, R₄, R₅ et R₇ sont tels que définis précédemment, composés de formule (IX) qui sont oxydés au moyen d'un oxydant couramment utilisé en synthèse organique,
      pour conduire aux composés de formule (**I/k**), cas particulier des composés de formule (I): 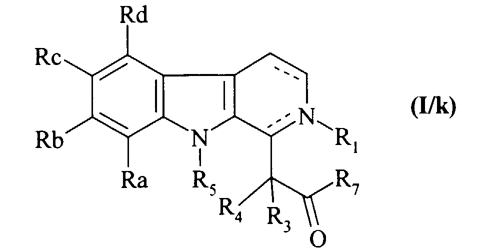 dans laquelle Ra, Rb, Rc, Rd, R₁, R₃, R₄, R₅ et R₇ sont tels que définis précédemment,
   ◆ *soit* que l'on traite par du diphénylphosphoryle azide, en présence de triéthylamine et d'un composé de formule (VI) R₇-OH, tel que défini précédemment, pour conduire aux composés de formule (**I/l**), cas particulier des composés de formule (I): 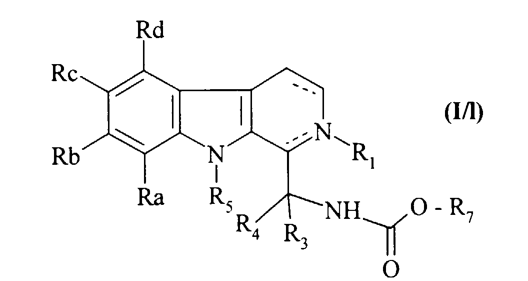 dans laquelle Ra, Rb, Rc, Rd, R₁, R₃, R₄, R₅ et R₇ sont tels que définis précédemment, composés de formule (I/1) dans le cas particulier où R7 représente un groupement benzyle, que l'on soumet à des conditions d'hydrogénolyse en présence de palladium sur charbon, pour conduire aux composés de formule (**I/m**), cas particulier des composés de formule (I): 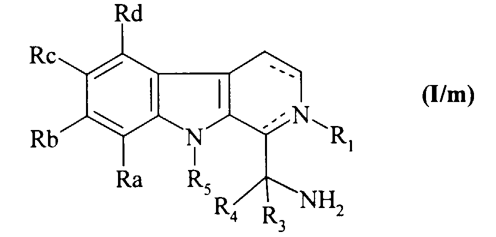 composés de formule (I/m) dont la fonction amine primaire est transformée en fonction amine secondaire ou tertiaire, selon les méthodes classiques, pour conduire aux composés de formule (**I/n**), cas particulier des composés de formule (I): 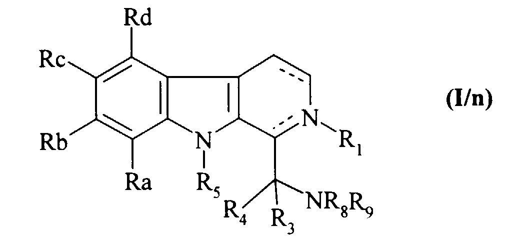 dans laquelle Ra, Rb, Rc, Rd, R₁, R₃, R₄, R₅, R₈ et R₉ ont la même définition que dans la formule (I), étant entendu que, dans ce cas, R₈ et R₉ ne représentent pas simultanément un atome d'hydrogène,
les composés (I/a) à (I/n) formant l'ensemble des composés de l'invention, que l'on purifie, le cas échéant, selon une technique classique de purification, qui peuvent, si on le désire, être séparés en leurs différents isomères, selon une technique classique de séparation, et que l'on transforme, le cas échéant, en leurs sels d'addition à un acide ou à une base pharmaceutiquement acceptable.

Les composés de formule (II), (III), (V), (VI), (VII) et (VIII) sont soit des composés commerciaux soit obtenus selon des méthodes connues de la synthèse organique.

La présente invention a également pour objet les compositions pharmaceutiques renfermant comme principe actif au moins un composé de formule (I), ses isomères optiques ou un de ses sels d'addition à un acide ou à une base pharmaceutiquement acceptables, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les sachets, les gélules, les tablettes, les suppositoires, les crèmes, les pommades, les gels dermiques, les préparations injectables ou buvables, les aérosols, les gouttes oculaires ou nasales, etc...

Les composés de l'invention présentent une forte activité sérotoninergique 5HT_{2B}/5HT_{2C} et notamment antagonistes 5-HT_{2C} (mises en évidence lors de test de liaison, où lesdits composés de l'invention possèdent notamment pour ce récepteur un Kᵢ compris entre 10⁻⁷ et 10⁻⁹ nM). Les compositions pharmaceutiques contenant au moins un composés de formule (I) sont utiles, de ce fait, dans le traitement de la dépression, de la psychose, de la schizophrénie, de la phobie, de l'anxiété, des attaques de panique, des troubles du sommeil, des troubles de l'appétit, des troubles impulsifs et agressifs, des troubles sexuels, et de la migraine.

La posologie utile varie selon l'âge et le poids du patient, la voie d'administration, la nature et la sévérité de l'affection, et la prise de traitements éventuels, et s'échelonne de 0,1 mg à 500 mg en une ou plusieurs prises par jour.

Les exemples suivants illustrent l'invention mais ne la limitent en aucune façon.

Les produits de départ et/ou les réactifs utilisés sont des produits connus ou préparés selon des modes opératoires connus.
Les structures des composés décrits dans les exemples et les stades de synthèse ont été déterminées selon les techniques spectrophotométriques usuelles (infrarouge, RMN, spectométrie de masse, ...).

### EXEMPLE 1 : Chlorhydrate de 1-(6-chloro-2,3,4,9-tétrahydro-1H-β-carbolin-1-yl) cyclobutanecarboxylate d'éthyle

### Stade A : 1-({[2-(5-Chloro-1H-indol-3-yl)éthyl]amino}carbonyl)cyclobutanecarboxylate d'éthyle

23,1 g de chlorhydrate de 5-chlorotryptamine, 17,3 g de cyclobutanedicarboxylate de monoéthyle, 36 ml de diisopropyléthylamine et 33,7 g de O-(benzotriazol-1-yl)-N,N,N',N'-tétraméthyluronium tétrafluoroborate sont agités 20 heures à température ambiante, dans 200 ml de dichlorométhane. Après lavage à l'eau, séchage sur sulfate de sodium, filtration sur célite de la phase organique, une évaporation sous pression réduite permet d'isoler le produit attendu.

### Stade B : Chlorhydrate de 1-(6-chloro-2,3,4,9-tétrahydro-1H-β-carbolin-1-yl) cyclobutanecarboxylate d'éthyle

32 g du produit obtenu au stade A sont portés à reflux dans une solution de 400 ml de toluène et de 35 ml de POCl₃. Après 3 heures, le milieu réactionnel est concentré sous pression réduite et le résidu est repris par 300 ml d'éthanol et 5 g de borohydrure de sodium sont additionnés lentement. Après 30 minutes, 300 ml d'eau sont additionnés et l'éthanol est distillé. Après extraction du résidu au dichlorométhane, séchage et évaporation sous pression réduite, le résidu huileux obtenu est transformé en chlorhydrate dans une solution d'éthanol chlorhydrique. Après filtration et séchage sous vide, on isole le produit attendu. *Point de fusion : 158-160°C*

| *Microanalyse élémentaire* : | | | | |
|---|---|---|---|---|
| | *C* | *H* | *N* | *Cl* |
| *% calculé* | *58, 54* | *6,00* | *7,59* | *19,20* |
| *% trouvé* | *58, 33* | *5,97* | *7,61* | *19,60* |

### EXEMPLE 2 : (R,S)-Chlorhydrate de 1-(6-bromo-2,3,4,9-tétrahydro-1H-β-carbolin-1-yl)cyclobutanecarboxylate d'éthyle

On procède comme dans l'exemple 1 en utilisant comme substrat au stade A le chlorhydrate de 5-bromotryptamine.
*Point de fusion : 246-247°C*

| *Microanalyse élémentaire* : | | | |
|---|---|---|---|
| | *C* | *H* | *N* |
| *% calculé* | *52,25* | *5,36* | *6,77* |
| *% trouvé* | *52,14* | *5,41* | *6,61* |

### EXEMPLE 3 : (+)-1-(6-Bromo-2,3,4,9-tétrahydro-1H-β-carbolin-1-yl)cyclobutane-carboxylate d'éthyle et son chlorhydrate

Le produit de l'exemple 2 est soumis à une chromatographie sur phase chirale (Chiralcel AD), la phase mobile étant constituée d'un mélange éthanol/diéthylamine : 1000/1. Le composé est élué avec un excès énantiomérique de 98 %, puis est transformé en chlorhydrate par action de l'éther chlorhydrique.
*Point de fusion : 226-227°C*
[α]_{D}^{21°C} = + 23,26°

### EXEMPLE 4 : (-)-1-(6-Bromo-2,3,4,9-tétrahydro-1H-β-carbolin-1-yl)cyclobutane carboxylate d'éthyle et son chlorhydrate

L'autre produit séparé au cours de la chromatographie mise en oeuvre à l'exemple 3 correspond au produit attendu, avec un excès énantiomérique de 95 %, celui-ci est ensuite transformé en son chlorhydrate.
*Point de fusion : 226-227°C*
[α]_{D}^{21°C} = -23,18°

### EXEMPLE 5 : Chlorhydrate de 1-(6-méthyl-2,3,4,9-tétrahydro-1H-)-carbolin-1-yl) cyclobutanecarboxylate d'éthyle

On procède comme dans l'exemple 1 en utilisant comme substrat au stade A le chlorhydrate de 5-méthyltryptamine.
*Point de fusion : 235-237°C*

| *Microanalyse élémentaire* : | | | | |
|---|---|---|---|---|
| | *C* | *H* | *N* | *Cl* |
| *% calculé* | *65,41* | *7,22* | *8,03* | *10,16* |
| *% trouvé* | *65,27* | *7,29* | *7,88* | *10,46* |

### EXEMPLE 6 : Chlorhydrate de 1-(6,7-dichloro-2,3,4,9-tétrahydro-1H-β-carbolin-1-yl)cyclobutanecarboxylate d'éthyle

On procède comme dans l'exemple 1 en utilisant comme substrat au stade A le chlorhydrate de 5,6-dichlorotryptamine.
*Point de fusion : > 260°C*

| *Microanalyse élémentaire* : | | | | |
|---|---|---|---|---|
| | *C* | *H* | *N* | *Cl* |
| *% calculé* | *53,55* | *5,24* | *6,94* | *26,34* |
| *% trouvé* | *53,88* | *4,99* | *6,83* | *26,10* |

### EXEMPLE 7 : Chlorhydrate de 1-(6-méthoxy-2,3,4,9-tétrahydro-1H-β-carbolin-1-yl) cyclobutanecarboxylate d'éthyle

On procède comme dans l'exemple 1 en utilisant comme substrat au stade A le chlorhydrate de 5-méthoxytryptamine.
*Point de fusion : 190-192°C*

| *Microanalyse élémentaire* : | | | | |
|---|---|---|---|---|
| | *C* | *H* | *N* | *Cl* |
| *% calculé* | *62,55* | *6,91* | *7,68* | *9,72* |
| *% trouvé* | *62,07* | *7,10* | *7,47* | *9,82* |

### EXEMPLE 8 : Chlorhydrate de 1-(2,3,4,9-tétrahydro-1H-β-carbolin-1-yl)cyclobutane carboxylate d'éthyle

On procède comme dans l'exemple 1 en utilisant comme substrat au stade A le chlorhydrate de tryptamine.
*Point de fusion : 204-206°C*

| *Microanalyse élémentaire* : | | | | |
|---|---|---|---|---|
| | *C* | *H* | *N* | *Cl* |
| *% calculé* | *64,57* | *6,92* | *8,37* | *10,59* |
| *% trouvé* | *64,60* | *6,98* | *8,25* | *10,66* |

### EXEMPLE 9 : Chlorhydrate de 1-(6,7-diméthoxy-2,3,4,9-tétrahydro-1H-β-carbolin-1-yl)cyclobutanecarboxylate d'éthyle

On procède comme dans l'exemple 1 en utilisant comme substrat au stade A le chlorhydrate de 5,6-diméthoxytryptamine.
*Point de fusion : 169-170°C*

| *Microanalyse élémentaire* : | | | | |
|---|---|---|---|---|
| | *C* | *H* | *N* | *Cl* |
| *% calculé* | *60,83* | *6,89* | *7,09* | *8,98* |
| *% trouvé* | *60,22* | *7,00* | *7,05* | *8, 66* |

### EXEMPLE 10 : Chlorhydrate de 1-(6,7-dibromo-2,3,4,9-tétrahydro-1H-β-carbolin-1-yl)cyclobutanecarboxylate d'éthyle

On procède comme dans l'exemple 1 en utilisant comme substrat au stade A le chlorhydrate de 5,6-dibromotryptamine.
*Point de fusion : > 260°C*

| *Microanalyse élémentaire* : | | | | | |
|---|---|---|---|---|---|
| | *C* | *H* | *N* | *Cl* | *Br* |
| *% calculé* | *43,89* | *4,30* | *5,69* | *7,20* | *32,44* |
| *% trouvé* | *44,00* | *4,31* | *5,67* | *7,29* | *32,00* |

### EXEMPLE 11 : Chlorhydrate de 1-(6-phényl-2,3,4,9-tétrahydro-1H-β-carbolin-1-yl) cyclobutanecarboxylate d'éthyle

2 g du composé obtenu dans l'exemple 2, 1 g d'acide phénylboronique, 1,7 g de fluorure de césium, 3,2 g de bromure de césium et 0,7 g de tétrakistriphénylphosphine palladium (0) sont portés 20 heures à reflux dans 200 ml de diméthoxyéthane, puis purifiés sur gel de silice (dichlorométhane/méthanol : 97/3) pour conduire, après salification par une solution d'éther chlorhydrique, au composé attendu.
*Point de fusion : > 260°C*

| *Microanalyse élémentaire* : | | | | |
|---|---|---|---|---|
| | *C* | *H* | *N* | *Cl* |
| *% calculé* | *69,31* | *6,67* | *6,74* | *8,53* |
| *% trouvé* | *68,70* | *6,45* | *6,65* | *8,27* |

### EXEMPLE 12 : Chlorhydrate de 1-(7-trifluorométhyl-2,3,4,9-tétrahydro-1H-β-carbolin-1-yl)cyclobutanecarboxylate d'éthyle

On procède comme dans l'exemple 1 en utilisant comme substrat au stade A, la 6-trifluorométhyltryptamine.
*Point de fusion : 241°C*

| *Microanalyse élémentaire* : | | | | |
|---|---|---|---|---|
| | *C* | *H* | *N* | *Cl* |
| *% calculé* | *56,65* | *5,50* | *6,95* | *8,80* |
| *% trouvé* | *56,68* | *5,29* | *6,82* | *9,10* |

### EXEMPLE 13 : Chlorhydrate de 1-(6-tert-butyl-2,3,4,9-tétrahydro-1H-β-carbolin-1-yl)cyclobutanecarboxylate d'éthyle

On procède comme dans l'exemple 1 en utilisant comme substrat au stade A le chlorhydrate de 5-tert-butyltryptamine.
*Point de fusion : lyophilisat*

| *Microanalyse élémentaire* : | | | | |
|---|---|---|---|---|
| | *C* | *H* | *N* | *Cl* |
| *% calculé* | *64,68* | *8,12* | *6,86* | *8,68* |
| *% trouvé* | *64,20* | *8,16* | *7,07* | *8,90* |

### EXEMPLE 14 : Chlorhydrate de 1-(7-méthyl-2,3,4,9-tétrahydro-1H-β-carbolin-1-yl) cyclobutanecarboxylate d'éthyle

On procède comme dans l'exemple 1 en utilisant comme substrat au stade A le chlorhydrate de 6-méthyltryptamine.
*Point de fusion : 236°C*

| *Microanalyse élémentaire* : | | | | |
|---|---|---|---|---|
| | *C* | *H* | *N* | *Cl* |
| *% calculé* | *64,49* | *7,60* | *7,92* | *10,02* |
| *% trouvé* | *64,05* | *7,86* | *7,73* | *10,20* |

### EXEMPLE 15 : 1-(6-Hydroxy-2,3,4,9-tétrahydro-1H-β-carbolin-1-yl)cyclobutane carboxylate d'éthyle

A une solution de 2 g du composé obtenu dans l'exemple 7 dans 50 ml de dichlorométhane, sont additionnés, à -30°C et sous atmosphère inerte, 12 ml de tribromure de bore 1M dans le dichlorométhane. Après 2 heures de réaction à température ambiante, le milieu réactionnel est hydrolysé par 1 ml d'une solution saturée de chlorure d'ammonium. Le précipité obtenu est filtré, rincé à l'eau puis séché permettant d'isoler le produit attendu. *Point de fusion : 211-216°C*

| *Microanalyse élémentaire* : | | | |
|---|---|---|---|
| | *C* | *H* | *N* |
| *% calculé* | *68,77* | *7,05* | *8,91* |
| *% trouvé* | *67,56* | *6,88* | *8,77* |

### EXEMPLE 16 : Chlorhydrate de 1-(7-chloro-6-fluoro-2,3,4,9-tétrahydro-1H-β-carbolin-1-yl)cyclobutanecarboxylate d'éthyle

On procède comme dans l'exemple 1 en utilisant comme substrat au stade A le 5-fluoro-6-chlorotryptamine.
*Point de fusion : 251-252°C*

| *Microanalyse élémentaire* : | | | | |
|---|---|---|---|---|
| | *C* | *H* | *N* | *Cl* |
| *% calculé* | *58,54* | *6,00* | *7,59* | *19,20* |
| *% trouvé* | *58,33* | *5,97* | *7,61* | *19,60* |

### EXEMPLE 17 : Chlorhydrate de 1-(6-fluoro-2,3,4,9-tétrahydro-1H-β-carbolin-1-yl) cyclobutanecarboxylate d'éthyle

On procède comme dans l'exemple 1 en utilisant comme substrat au stade A la 5-fluorotryptamine.
*Point de fusion : 212-213°C*

| *Microanalyse élémentaire* : | | | | |
|---|---|---|---|---|
| | *C* | *H* | *N* | *Cl* |
| *% calculé* | *61,27* | *6,28* | *7,94* | *10,05* |
| *% trouvé* | *61,25* | *6,28* | *7,91* | *10,38* |

### EXEMPLE 18 : Chlorhydrate de 1-(5,6-dichioro-2,3,4,9-tétrahydro-1H-β-carbolin-1-yl)cyclobutanecarboxylate d'éthyle

On procède comme dans l'exemple 1 en utilisant comme substrat au stade A le chlorhydrate de 4,5-dichlorotryptamine.
*Point de fusion : 242-243°C*

| *Microanalyse élémentaire* : | | | | |
|---|---|---|---|---|
| | *C* | *H* | *N* | *Cl* |
| *% calculé* | *53,58* | *5,33* | *6,87* | *26,07* |
| *% trouvé* | *53,49* | *5,41* | *6,72* | *26,59* |

### EXEMPLE 19 : Chlorhydrate de 1-(5,6-dibromo-2,3,4,9-tétrahydro-1H-β-carbolin-1-yl)cyclobutanecarboxylate d'éthyle

On procède comme dans l'exemple 1 en utilisant comme substrat au stade A le chlorhydrate de 4,5-dibromotryptamine.
*Point de fusion : 183-184°C*

| *Microanalyse élémentaire* : | | | | | |
|---|---|---|---|---|---|
| | *C* | *H* | *N* | *Cl* | *Br* |
| *% calculé* | *43,89* | *4,30* | *5,69* | *7,20* | *32,44* |
| *% trouvé* | *44,20* | *4,40* | *5,69* | *7,30* | *32,00* |

### EXEMPLE 20 : Chlorhydrate de 1-(6-chloro-9-méthyl-2,3,4,9-tétrahydro-1H-β-carbolin-1-yl)cyclobutanecarboxylate d'éthyle

On procède comme dans l'exemple 1 en utilisant comme substrat au stade A le chlorhydrate de 5-chloro-1-méthyltryptamine.
*Point de fusion : > 260°C*

| *Microanalyse élémentaire* : | | | | |
|---|---|---|---|---|
| | *C* | *H* | *N* | *Cl* |
| *% calculé* | *59,54* | *6,31* | *7,31* | *18,50* |
| *% trouvé* | *59,80* | *6,39* | *7,09* | *18,38* |

### EXEMPLE 21 : Chlorhydrate de 1-(6-chloro-9-éthyl-2,3,4,9-tétrahydro-1H-β-carbolin-1-yl)cyclobutanecarboxylate d'éthyle

On procède comme dans l'exemple 1 en utilisant comme substrat au stade A la 5-chloro-1-éthyltryptamine.
*Point de fusion : 231-232°C*

| *Microanalyse élémentaire* : | | | | |
|---|---|---|---|---|
| | *C* | *H* | *N* | *Cl* |
| *% calculé* | *60,46* | *6,60* | *7,05* | *17,84* |
| *% trouvé* | *60,48* | *6,73* | *7,02* | *18,11* |

### EXEMPLE 22 : Chlorhydrate de 1-(6-méthoxy-9-méthyl-2,3,4,9-tétrahydro-1H-β-carbolin-1-yl)cyclobutanecarboxylate d'éthyle

On procède comme dans l'exemple 1 en utilisant comme substrat au stade A le chlorhydrate de 5-méthoxy-1-méthyltryptamine.
*Point de fusion : > 250°C*

| *Microanalyse élémentaire* : | | | | |
|---|---|---|---|---|
| | *C* | *H* | *N* | *Cl* |
| *% calculé* | *63,40* | *7,18* | *7,39* | *9,36* |
| *% trouvé* | *62,96* | *7,62* | *7,17* | *9,06* |

### EXEMPLE 23 : Chlorhydrate de 1-(6-chloro-2,3,4,9-tétrahydro-1H-β-carbolin-1-yl)cyclobutanecarboxylate de cyclopentyle

4,0 g du composé de l'exemple 1 sont portés au reflux de 80 ml de cyclopentanol, en présence de 1 ml de tétraisopropoxyde de titane. Après 24 heures de réaction, le milieu réactionnel est dilué à l'eau puis filtré. Après extraction au dichlorométhane, séchage et évaporation sous pression réduite, une cristallisation du résidu dans un milieu éthanol/éther éthylique chlorhydrique permet d'isoler le produit attendu.
*Point de fusion : 208-209°C*

| *Microanalyse élémentaire* : | | | | |
|---|---|---|---|---|
| | *C* | *H* | *N* | *Cl* |
| *% calculé* | *61,62* | *6,40* | *6,84* | *17,32* |
| *% trouvé* | *61,42* | *6,50* | *6,65* | *17,17* |

### EXEMPLE 24 : Chlorhydrate de 1-(6-chloro-2,3,4,9-tétrahydro-1H-β-carbolin-1-yl)cyclobutanecarboxylate d'isopropyle

On procède comme dans l'exemple 23 en utilisant comme réactif l'isopropanol à la place du cyclopentanol.
*Point de fusion : 244-245°C*

| *Microanalyse élémentaire* : | | | | |
|---|---|---|---|---|
| | *C* | *H* | *N* | *Cl* |
| *% calculé* | *59,54* | *6,31* | *7,31* | *18,50* |
| *% trouvé* | *59,5* | *6,29* | *7,19* | *18,72* |

### EXEMPLE 25 : Chlorhydrate de 1-(6-méthoxy-2,3,4,9-tétrahydro-1H-β-carbolin-1-yl)cyclobutanecarboxylate de benzyle

On procède comme dans l'exemple 23 en utilisant comme substrat le produit de l'exemple 7 et comme réactif l'alcool benzylique.
*Point de fusion : 228-230°C*

| *Microanalyse élémentaire* : | | | | |
|---|---|---|---|---|
| | *C* | *H* | *N* | *Cl* |
| *% calculé* | *67,52* | *6,37* | *6,56* | *8,30* |
| *% trouvé* | *67,96* | *6,49* | *6,64* | *8,77* |

### EXEMPLE 26 : Chlorhydrate de 1-(6-chloro-2,3,4,9-tétrahydro-1H-β-carbolin-1-yl) cyclobutanecarboxylate de méthyle

On procède comme dans l'exemple 23 en utilisant comme réactif le méthanol. *Point de fusion : 232-233°C*

| *Microanalyse élémentaire* : | | | | |
|---|---|---|---|---|
| | *C* | *H* | *N* | *Cl* |
| *% calculé* | *57,07* | *5,71 71* | *7,83* | *19,82* |
| *% trouvé* | *56,99* | *5,54* | *7,50* | *20,27* |

### EXEMPLE 27 : Chlorhydrate de 1-(6-méthoxy-2,3,4,9-tétrahydro-1H-β-carbolin-1-yl)cyclobutanecarboxylate de méthyle

On procède comme dans l'exemple 23 à partir du composé de l'exemple 7 et du réactif de l'exemple 26.
*Point de fusion : 208-209°C*

| *Microanalyse élémentaire* : | | | | |
|---|---|---|---|---|
| | *C* | *H* | *N* | *Cl* |
| *% calculé* | *61,62* | *6,61* | *7,98* | *10,11* |
| *% trouvé* | *61,56* | *6,61* | *7,88* | *10,23* |

### EXEMPLE 28 : Chlorhydrate de 1-(6-chloro-2,3,4,9-tétraydro-1H-β-carbolin-1-yl) cyclohexanecarboxylate d'éthyle

On procède comme dans l'exemple 1 en utilisant comme réactif le cyclohexanedicarboxylate de monoéthyle.
*Point de fusion : 245-246°C*

| *Microanalyse élémentaire* : | | | | |
|---|---|---|---|---|
| | *C* | *H* | *N* | *Cl* |
| *% calculé* | *60,46* | *6,60* | *7,05* | *17,84* |
| *% trouvé* | *60,30* | *6,52* | *7,01* | *18,24* |

### EXEMPLE 29 : Chlorhydrate de 1-(6-chloro-2,3,4,9-tétrahydro-1H-β-carbolin-1-yl) cyclopentanecarboxylate d'éthyle

On procède comme dans l'exemple 1 en utilisant comme réactif le cyclopentanedicarboxylate de monométhyle.
*Point de fusion : 231-232°C*

| *Microanalyse élémentaire* : | | | | |
|---|---|---|---|---|
| | *C* | *H* | *N* | *Cl* |
| *% calculé* | *59,54* | *6,31* | *7,31* | *18,50* |
| *% trouvé* | *59,63* | *6,32* | *7,08* | *18,63* |

### EXEMPLE 30 : Chlorhydrate de 1-(6-chloro-9-méthyl-2,3,4,9-tétrahydro-1H-β-carbolin-1-yl)cyclopentanecarboxylate d'éthyle

On procède comme dans l'exemple 20 en utilisant comme réactif au stade A celui utilisé dans l'exemple 29.
*Point de fusion : 113-115°C*

| *Microanalyse élémentaire* : | | | | |
|---|---|---|---|---|
| | *C* | *H* | *N* | *Cl* |
| *% calculé* | *66,56* | *6,98* | *7,76* | *9,82* |
| *% trouvé* | *66,23* | *7,16* | *7,49* | *10,28* |

### EXEMPLE 31 : Chlorhydrate de 1-(6-méthoxy-2,3,4,9-tétrahydro-1H-β-carbolin-1-yl)cyclopentanecarboxylate d'éthyle

On procède comme dans l'exemple 7 en utilisant comme réactif au stade A celui utilisé dans l'exemple 29.
*Point de fusion : 175-176°C*

| *Microanalyse élémentaire* : | | | | |
|---|---|---|---|---|
| | *C* | *H* | *N* | *Cl* |
| *% calculé* | *63,02* | *7,21* | *7,34* | *9,30* |
| *% trouvé* | *62,72* | *7,43* | *7,17* | *9,14* |

### EXEMPLE 32 : Acide 1-(6-chloro-2,3,4,9-tétrahydro-1H-β-carbolin-1-yl) cyclobutanecarboxylique

Une suspension de 1 g du composé de l'exemple 1 dans 15 ml d'eau, 30 ml d'éthanol et 5 ml de soude 1M, est chauffée à 50°C pendant 10 heures. Le mélange réactionnel est ensuite refroidi et 5 ml d'HCl 1M sont additionnés. Le précipité formé est essoré, rincé à l'eau puis séché sous vide et permet d'isoler le produit attendu.
*Point de fusion : > 275°C*

| *Microanalyse élémentaire* : | | | | |
|---|---|---|---|---|
| | *C* | *H* | *N* | *Cl* |
| *% calculé* | *63,05* | *5,62* | *9,19* | *11,63* |
| *% trouvé* | *62,22* | *5,67* | *9,20* | *11,75* |

### EXEMPLE 33 : Acide 1-(6-chloro-9-méthyl-2,3,4,9-tétrahydro-1H-β-carbolin-1-yl) cyclobutanecarboxylique

On procède comme dans l'exemple 32 en utilisant comme substrat le composé obtenu dans l'exemple 20.
*Point de fusion : > 260°C*

| *Microanalyse élémentaire* : | | | | |
|---|---|---|---|---|
| | *C* | *H* | *N* | *Cl* |
| *% calculé* | *64,05* | *6,01* | *8,79* | *11,12* |
| *% trouvé* | *64,08* | *5,95* | *8,67* | *11,35* |

### EXEMPLE 34 : Acide 1-(6-chloro-2,3,4,9-tétrahydro-1H-β-carbolin-1-yl) cyclopentanecarboxylique

On procède comme dans l'exemple 32 en utilisant comme substrat le composé obtenu dans l'exemple 29.
*Point de fusion : > 260°C*

| *Microanalyse élémentaire* : | | | | |
|---|---|---|---|---|
| | *C* | *H* | *N* | *Cl* |
| *% calculé* | *64,05* | *6,01* | *8,79* | *11,12* |
| *% trouvé* | *64,45* | *6,13* | *8,66* | *11,50* |

### EXEMPLE 35 : Acide 1-(2,3,4,9-tétrahydro-1H-β-carbolin-1-yl) cyclobutanecarboxylique

On procède comme dans l'exemple 32 en utilisant comme substrat le composé obtenu dans l'exemple 8.
*Point de fusion : 280-281 °C*

| *Microanalyse élémentaire* : | | | |
|---|---|---|---|
| | *C* | *H* | *N* |
| *% calculé* | *71,09* | *6,71* | *10,36* |
| *% trouvé* | *71,11* | *6,88* | *10,22* |

### EXEMPLE 36 : Acide 1-(6-méthoxy-2,3,4,9-tétrahydro-1H-β-carbolin-1-yl) cyclobutanecarboxylique

On procède comme dans l'exemple 32 en utilisant comme substrat le composé obtenu dans l'exemple 7.
*Point de fusion : > 260°C*

| *Microanalyse élémentaire* : | | | |
|---|---|---|---|
| | *C* | *H* | *N* |
| *% calculé* | *67,98* | *6,71* | *9,33* |
| *% trouvé* | *67,96* | *7,03* | *8,96* |

### EXEMPLE 37 : Acide 1-(6-méthyl-2,3,4,9-tétrahydro-1H-β-carbolin-1-yl) cyclobutanecarboxylique

On procède comme dans l'exemple 32 en utilisant comme substrat le composé de l'exemple 5.
*Point de fusion : > 250°C*

| Microanalyse élémentaire : | | | |
|---|---|---|---|
| | *C* | *H* | *N* |
| *% calculé* | *71,37* | *7,11* | *9,79* |
| *% trouvé* | *70,98* | *7,15* | *9,58* |

### EXEMPLE 38 : Acide 1-(7-méthyl-2,3,4,9-tétrahydro-1H-β-carbolin-1-yl) cyclobutanecarboxylique

On procède comme dans l'exemple 32 en utilisant comme substrat le composé de l'exemple 14.
*Point de fusion : > 260°C*

| *Microanalyse élémentaire* : | | | |
|---|---|---|---|
| | *C* | *H* | *N* |
| *% calculé* | *69,58* | *6,98* | *9,54* |
| *% trouvé* | *70,10* | *7,10* | *9,54* |

### EXEMPLE 39 : Acide 1-(6-tert-butyl-2,3,4,9-tétrahydro-1H-β-carbolin-1-yl) cyclobutanecarboxylique

On procède comme dans l'exemple 32 en utilisant comme substrat le composé de l'exemple 13.
*Point de fusion : > 260°C*

| *Microanalyse élémentaire* : | | | |
|---|---|---|---|
| | *C* | *H* | *N* |
| *% calculé* | *70,13* | *8,27* | *8,18* |
| *% trouvé* | *70,6* | *7,98* | *8,29* |

### EXEMPLE 40 : Acide 1-(7-trifluorométhyl-2,3,4,9-tétrahydro-1H-β-carbolin-1-yl) cyclobutanecarboxylique

On procède comme dans l'exemple 32 en utilisant comme substrat le composé de l'exemple 12. *Point de fusion : > 260°C*

| *Microanalyse élémentaire* : | | | |
|---|---|---|---|
| | *C* | *H* | *N* |
| *% calculé* | *57,33* | *5,36* | *7,87* |
| *% trouvé* | *58,28* | *5,56* | *7,86* |

### EXEMPLE 41 : Chlorhydrate de 1-(6-chloro-2-méthyl-2,3,4,9-tétrahydro-1H-β-carbolin-1-yl)cyclobutanecarboxylate d'éthyle

A une solution de 1 g du composé de l'exemple 1 dans 25 ml d'éthanol sont additionnés 0,3 ml d'iodure de méthyle et 0,8 g de NaHCO₃. Après 24 heures de réaction à température ambiante, le milieu réactionnel est concentré sous pression réduite, puis le résidu est repris par un mélange eau et dichlorométhane. Après extraction, séchage et filtration, la phase organique est concentrée sous pression réduite. Une cristallisation dans une solution d'éthanol et d'éther diéthylique chlorhydrique permet d'obtenir le produit attendu. *Point de fusion : 170-171 °C*

| *Microanalyse élémentaire* : | | | | |
|---|---|---|---|---|
| | *C* | *H* | *N* | *Cl* |
| *% calculé* | *59,54* | *6,31* | *7,31* | *18,50* |
| *% trouvé* | *59,28* | *6,24* | *7,12* | *18,42* |

### EXEMPLE 42 : Chlorhydrate de 1-(6-chloro-2,9-diméthyl-2,3,4,9-tétrahydro-1H-β-carbolin-1-yl)cyclobutanecarboxylate d'éthyle

On procède comme dans l'exemple 41 en utilisant comme substrat le composé de l'exemple 20.
*Point de fusion : 238-240°C*

| *Microanalyse élémentaire* : | | | | |
|---|---|---|---|---|
| | *C* | *H* | *N* | *Cl* |
| *% calculé* | *60,46* | *6,60* | *7,05* | *17,84* |
| *% trouvé* | *60,49* | *6,57* | *6,90* | *18,25* |

### EXEMPLE 43 : 1-(6-Chloro-2-méthyl-2,3,4,9-tétrahydro-1H-β-carbolin-1-yl) cyclopentanecarboxylate d'éthyle

On procède comme dans l'exemple 41 en utilisant comme substrat le composé de l'exemple 29.
*Point de fusion : 145-146°C*

| *Microanalyse élémentaire* : | | | | |
|---|---|---|---|---|
| | *C* | *H* | *N* | *Cl* |
| *% calculé* | *59,54* | *6,31* | *7,31* | *18,50* |
| *% trouvé* | *59,28* | *6,24* | *7,12* | *18,42* |

### EXEMPLE 44 : 1-(6-Chloro-2,9-diméthyl-2,3,4,9-tétrahydro-1H-β-carbolin-1-yl) cyclopentanecarboxylate d'éthyle

On procède comme dans l'exemple 41 en utilisant comme substrat le composé de l'exemple 30.
*Point de fusion : 109°C*

| *Microanalyse élémentaire* : | | | | |
|---|---|---|---|---|
| | *C* | *H* | *N* | *Cl* |
| *% calculé* | *67,28* | *7,26* | *7,47* | *9,46* |
| *% trouvé* | *67,26* | *7,46* | *7,56* | *10,19* |

### EXEMPLE 45 : Chlorhydrate de 1-(6-chloro-2-méthyl-2,3,4,9-tétrahydro-1H-β-carbolin-1-yl)cyclohexanecarboxylate d'éthyle

On procède comme dans l'exemple 41 en utilisant comme substrat le composé de l'exemple 28.
*Point de fusion : 178-185°C*

| *Microanalyse élémentaire* : | | | | |
|---|---|---|---|---|
| | *C* | *H* | *N* | *Cl* |
| *% calculé* | *61,31* | *6,86* | *6,81* | *17,24* |
| *% trouvé* | *61,36* | *6,77* | *6,71* | *16,89* |

### EXEMPLE 46 : 1-(2-Méthyl-2,3,4,9-tétrahydro-1H-β-carbolin-1-yl) cyclobutanecarboxylate d'éthyle

On procède comme dans l'exemple 41 en utilisant comme substrat le composé de l'exemple 8.
*Point de fusion : 142-144°C*

| *Microanalyse élémentaire* : | | | |
|---|---|---|---|
| | *C* | *H* | *N* |
| *% calculé* | *73,05* | *7,74* | *8,97* |
| *% trouvé* | *72,66* | *7,75* | *8,79* |

### EXEMPLE 47 : 1-(6-Méthoxy-2-méthyl-2,3,4,9-tétrahydro-1H-β-carbolin-1-yl) cyclobutanecarboxylate d'éthyle

On procède comme dans l'exemple 41 en utilisant comme substrat le composé de l'exemple 7.
*Point de fusion : 124-125°C*

| *Microanalyse élémentaire* : | | | |
|---|---|---|---|
| | *C* | *H* | *N* |
| *% calculé* | *70,15* | *7,65* | *8,18* |
| *% trouvé* | *70,16* | *7,64* | *8,00* |

### EXEMPLE 48 : 1-(6-Méthoxy-2-méthyl-2,3,4,9-tétrahydro-1H-β-carbolin-1-yl) cyclopentanecarboxylate d'éthyle

On procède comme dans l'exemple 41 en utilisant comme substrat le composé de l'exemple 31.
*Point de fusion : 113-114°C*

| *Microanalyse élémentaire* : | | | |
|---|---|---|---|
| | *C* | *H* | *N* |
| *% calculé* | *70,76* | *7,92* | *7,86* |
| *% trouvé* | *70,21* | *7,85* | *7,75* |

### EXEMPLE 49 : 1-(2-Benzyl-6-chloro-2,3,4,9-tétrahydro-1H-β-carbolin-1-yl) cyclobutanecarboxylate d'éthyle

On procède comme dans l'exemple 41 en utilisant le bromure de benzyle comme réactif à la place de l'iodure de méthyle.
*Point de fusion : 155-156°C*

| *Microanalyse élémentaire* : | | | | |
|---|---|---|---|---|
| | *C* | *H* | *N* | *Cl* |
| *% calculé* | *70,99* | *6,43* | *6,62* | *8,38* |
| *% trouvé* | *70,95* | *6,51* | *6,79* | *9,08* |

### EXEMPLE 50 : Acide 1-(6-chloro-2-méthyl-2,3,4,9-tétrahydro-1H-β-carbolin-1-yl) cyclobutanecarboxylique

On procède comme dans l'exemple 32 en utilisant comme substrat le composé de l'exemple 41.
*Point de fusion : 174-175°C*

| *Microanalyse élémentaire* : | | | | |
|---|---|---|---|---|
| | *C* | *H* | *N* | *Cl* |
| *% calculé* | *64,05* | *6,01* | 8,79 | 11,12 |
| *% trouvé* | *63,97* | *5,95* | *8,59* | *11,45* |

### EXEMPLE 51 : Acide 1-(6-chloro-2,9-diméthyl-2,3,4,9-tétrahydro-1H-β-carbolin-2-yl)cyclobutanecarboxylique

On procède comme dans l'exemple 32 en utilisant comme substrat le composé de l'exemple 42.
*Point de fusion : > 260°C*

| *Microanalyse élémentaire* : | | | | |
|---|---|---|---|---|
| | *C* | *H* | *N* | *Cl* |
| *% calculé* | *64,96* | *6,36* | *8,42* | *10,65* |
| *% trouvé* | *64,40* | *6,40* | *8,18* | *10,91* |

### EXEMPLE 52 : Acide 1-(6-chloro-2-méthyl-2,3,4,9-tétrahydro-1H-β-carbolin-1-yl) cyclopentanecarboxylique

On procède comme dans l'exemple 32 en utilisant comme substrat le composé de l'exemple 43.
*Point de fusion : 171-173°C*

| *Microanalyse élémentaire* : | | | | |
|---|---|---|---|---|
| | *C* | *H* | *N* | *Cl* |
| *% calculé* | *64,96* | *6,36* | *8,42* | *10,65* |
| *% trouvé* | *64,59* | *6,39* | *8,21* | *10,77* |

### EXEMPLE 53 : Acide 1-(6-chloro-2,9-diméthyl-2,3,4,9-tétrahydro-1H-β-carbolin-1-yl)cyclopentanecarboxylique

On procède comme dans l'exemple 32 en utilisant comme substrat le composé de l'exemple 44.
*Point de fusion : 197-198°C*

| *Microanalyse élémentaire* : | | | | |
|---|---|---|---|---|
| | *C* | *H* | *N* | *Cl* |
| *% calculé* | *62,54* | *6,91* | *7,68* | *9,71* |
| *% trouvé* | *62,83* | *7,07* | *7,64* | *9,99* |

### EXEMPLE 54 : Acide 1-(6-chloro-2-benzyl-2,3,4,9-tétrahydro-1H-β-carbolin-1-yl) cyclobutanecarboxylique

On procède comme dans l'exemple 32 en utilisant comme substrat le composé de l'exemple 49.
*Point de fusion : 178-180°C*

| *Microanalyse élémentaire* : | | | | |
|---|---|---|---|---|
| | *C* | *H* | *N* | *Cl* |
| *% calculé* | *68,00* | *6,01* | *6,89* | *8,72* |
| *% trouvé* | *67,81* | *6,04* | *6,91* | *9,30* |

### EXEMPLE 55 : Chlorhydrate de 1-[6-chloro-2-(1H-imidazol-5-ylcarbonyl)-2,3,4,9-tétrahydro-1H-β-carbolin-1-yl]cyclobutanecarboxylate d'éthyle

1,84 g du composé de l'exemple 1, 1,8 g d'acide N-trityl-imidazol-4-carboxylique, 1,92 ml de diisopropyléthylamine et 1,76 g de O-(benzotriazol-1-yl)-N,N,N',N'-tétraméthyluronium tétrafluoroborate sont agités à température ambiante pendant 24 heures. Après dilution par 500 ml d'eau, filtration, et extraction au dichlorométhane, les phases organiques sont séchées, filtrées, puis concentrées sous pression réduite. Une cristallisation du résidu dans un mélange éthanol/éther éthylique chlorhydrique permet d'isoler le composé attendu.
*Point de fusion : 170-171°C*

### EXEMPLE 56 : 1-(2-Benzoyl-6-chloro-2,3,4,9-tétrahydro-1H-β-carbolin-1-yl) cyclobutanecarboxylate d'éthyle

On procède comme dans l'exemple 55 en utilisant comme réactif le chlorure de benzoyle à la place de l'acide N-trityl-imidazol-4-carboxylique.
*Point de fusion : 195-196°C*

| *Microanalyse élémentaire* : | | | | |
|---|---|---|---|---|
| | *C* | *H* | *N* | *Cl* |
| *% calculé* | *68,72* | *5,77* | *6,41* | *8,11* |
| *% trouvé* | *68,36* | *5,80* | *6,26* | *8,07* |

### EXEMPLE 57 : 1-[6-Chloro-2-(2-thiénylcarbonyl)-2,3,4,9-tétrahydro-1H-β-carbolin-1-yl]cyclobutanecarboxylate d'éthyle

On procède comme dans l'exemple 55 en utilisant comme réactif le chlorure de 2-thiophénoyle.
*Point de fusion : 190-191°C*

| *Microanalyse élémentaire* : | | | | | |
|---|---|---|---|---|---|
| | *C* | *H* | *N* | *Cl* | *S* |
| *% calculé* | *62,36* | *5,23* | *6,32* | *7,24* | *8,00* |
| *% trouvé* | *62,43* | *5,29* | *6,30* | *7,17* | *7,98* |

### EXEMPLE 58 : 1-[6-Chloro-2-(3-furoyl)-2,3,4,9-tétrahydro-1H-β-carbolin-1-yl] cyclobutanecarboxylate d'éthyle

On procède comme dans l'exemple 55 en utilisant comme réactif le chlorure de 3-furoyle.
*Point de fusion : 176-177°C*

### EXEMPLE 59 : 1-[6-Chloro-2-(4-chlorobenzoyl)-2,3,4,9-tétrahydro-1H-β-carbolin-1-yl]cyclobutanecarboxylate d'éthyle

On procède comme dans l'exemple 55 en utilisant comme réactif le chlorure de 4-chlorobenzoyle.
*Point de fusion : 204-205°C*

| *Microanalyse élémentaire* : | | | | |
|---|---|---|---|---|
| | *C* | *H* | *N* | *Cl* |
| *% calculé* | *63,70* | *5,13* | *5,94* | *15,04* |
| *% trouvé* | *63,78* | *5,27* | *5,95* | *14,69* |

### EXEMPLE 60 : 1-[6-Chloro-2-(4-méthoxybenzoyl)-2,3,4,9-tétrahydro-1H-β-carbolin-1-yl]cyclobutanecarboxylate d'éthyle

On procède comme dans l'exemple 55 en utilisant comme réactif le chlorure de 4-méthoxybenzoyle.
*Point de fusion : 169-170°C*

| *Microanalyse élémentaire* : | | | | |
|---|---|---|---|---|
| | *C* | *H* | *N* | *Cl* |
| *% calculé* | *66,88* | *5,83* | *6,00* | *7,59* |
| *% trouvé* | *66,92* | *5,92* | *6,02* | *7,55* |

### EXEMPLE 61 : 1-[6-Bromo-2-(3-furoyl)-2,3,4,9-tétrahydro-1H-β-carbolin-1-yl] cyclobutanecarboxylate d'éthyle

On procède comme dans l'exemple 55 en utilisant comme substrat le composé de l'exemple 2 et comme réactif le chlorure de 3-furoyle.
*Point de fusion : 178-179°C*

| *Microanalyse élémentaire* : | | | | |
|---|---|---|---|---|
| | *C* | *H* | *N* | *Br* |
| *% calculé* | *58,61* | *4,92* | *5,94* | *16,95* |
| *% trouvé* | *58,61* | *5,11* | *5,77* | *16,92* |

### EXEMPLE 62 : 1-[6-Chloro-2-(2-pyridinylcarbonyl)-2,3,4,9-tétrahydro-1H-β-carbolin-1-yl]cyclobutanecarboxylate d'éthyle

On procède comme dans l'exemple 55 en utilisant comme réactif l'acide 2-pyridine carboxylique.
*Point de fusion : 166-167°C*

| *Microanalyse élémentaire* : | | | | |
|---|---|---|---|---|
| | *C* | *H* | *N* | *Cl* |
| *% calculé* | *65,83* | *5,52* | *9,60* | *8,10* |
| *% trouvé* | *65,71* | *5,67* | *9,58* | *8,29* |

### EXEMPLE 63 : 1-{6-Chloro-2-[(4-oxo-4H-pyran-2-yl)carbonyl]-2,3,4,9-tétrahydro-1H-β-carbolin-1-yl}cyclobutanecarboxylate d'éthyle

On procède comme dans l'exemple 55 en utilisant comme réactif l'acide 4-oxo-4*H*-pyran-3-carboxylique.
*Point de fusion : 153-154°C*

| *Microanalyse élémentaire* : | | | | |
|---|---|---|---|---|
| | *C* | *H* | *N* | *Cl* |
| *% calculé* | *63,37* | *5,10* | *6,16* | *7,79* |
| *% trouvé* | *63,38* | *5,17* | *6,08* | *7,96* |

### EXEMPLE 64 : 1-[6-Chloro-2-(3-pyridinylcarbonyl)-2,3,4,9-tétrahydro-1H-β-carbolin-1-yl]cyclobutanecarboxylate d'éthyle

On procède comme dans l'exemple 55 en utilisant comme réactif l'acide 3-pyridinecarboxylique.
*Point de fusion : 184-185°C*

| *Microanalyse élémentaire* : | | | | |
|---|---|---|---|---|
| | *C* | *H* | *N* | *Cl* |
| *% calculé* | *65,83* | *5,52* | *9,60* | *8,10* |
| *% trouvé* | *65,91* | *5,61* | *9,53* | *8,10* |

### EXEMPLE 65 : 1-[6-Chloro-2-(2-pyrazinylcarbonyl)-2,3,4,9-tétrahydro-1H-β-carbolin-1-yl]cyclobutanecarboxylate d'éthyle

On procède comme dans l'exemple 55 en utilisant comme réactif l'acide 2-pyrazinyl-carboxylique.
*Point de fusion : 167-168°C*

| *Microanalyse élémentaire* : | | | | |
|---|---|---|---|---|
| | *C* | *H* | *N* | *Cl* |
| *% calculé* | *62,94* | *5,28* | *12,76* | *8,08* |
| *% trouvé* | *63,04* | *5,40* | *12,86* | *8,03* |

### EXEMPLE 66 : 1-[6-Chloro-2-(2-pyrimidinylcarbonyl)-2,3,4,9-tétradydro-1H-β-carbolin-1-yl]cyclobutanecarboxylate d'éthyle

On procède comme dans l'exemple 55 en utilisant comme réactif l'acide 2-pyrimidinyl-carboxylique.
*Point de fusion : 158-160°C*

| *Microanalyse élémentaire* : | | | | |
|---|---|---|---|---|
| | *C* | *H* | *N* | *Cl* |
| *% calculé* | *62,94* | *5,28* | *12,76* | *8,08* |
| *% trouvé* | *62,04* | *5,30* | *12,72* | *8,11* |

### EXEMPLE 67 : 1-[6-Chloro-2-(5-isoxazolylcarbonyl)-2,3,4,9-tétrahydro-1H-β-carbolin-1-yl]cyclobutanecarboxylate d'éthyle

On procède comme dans l'exemple 55 en utilisant comme réactif l'acide 5-isoxazolylcarboxylique.
*Point de fusion : 166-167°C*

| *Microanalyse élémentaire* : | | | | |
|---|---|---|---|---|
| | *C* | *H* | *N* | *Cl* |
| *% calculé* | *61,76* | *5,18* | *9,82* | *8,29* |
| *% trouvé* | *61,80* | *5,24* | *9,74* | *8,33* |

### EXEMPLE 68 : 1-[6-Chloro-2-(4-pyridinylcarbonyl)-2,3,4,9-tétrahydro-1H-β-carbolin-1-yl]cyclobutanecarboxylate d'éthyle

On procède comme dans l'exemple 55 en utilisant comme réactif l'acide 4-pyridinecarboxylique.
*Point de fusion : 205-206°C*

| *Microanalyse élémentaire* : | | | | |
|---|---|---|---|---|
| | *C* | *H* | *N* | *Cl* |
| *% calculé* | *65,83* | *5,52* | *9,60* | *8,10* |
| *% trouvé* | *65,85* | *5,52* | *9,53* | *8,07* |

### EXEMPLE 69 : 1-[6-Bromo-2-(5-isoxazolylcarbonyl)-2,3,4,9-tétrahydro-1H-β-carbolin-1-yl]cyclobutanecarboxylate d'éthyle

On procède comme dans l'exemple 55 en utilisant comme substrat le composé de l'exemple 2 et comme réactif, celui de l'exemple 67.
*Point de fusion : 170-171 °C*

| *Microanalyse élémentaire* : | | | | |
|---|---|---|---|---|
| | *C* | *H* | *N* | *Br* |
| *% calculé* | *55,94* | *4,69* | *8,90* | *16,92* |
| *% trouvé* | *56,02* | *4,73* | *8,72* | *16,67* |

### EXEMPLE 70 : 1-[6-Chloro-2-(1H-pyrrol-2-ylcarbonyl)-2,3,4,9-tétrahydro-1H-β-carbolin-1-yl]cyclobutanecarboxylate d'éthyle

On procède comme dans l'exemple 55 en utilisant comme réactif l'acide 1*H*-2-pyrrole-carboxylique.
*Point de fusion : 209-210°C*

| *Microanalyse élémentaire* : | | | | |
|---|---|---|---|---|
| | *C* | *H* | *N* | *Cl* |
| *% calculé* | *64,86* | *5,68* | *9,87* | *8,32* |
| *% trouvé* | *64,89* | *5,73* | *9,76* | *8,48* |

### EXEMPLE 71 : 1-{6-Chloro-2-[2-(1H-1,2,3,4-tétraazol-1-yl)acétyl]-2,3,4,9-tétra hydro-1H-β-carbolin-1-yl}cyclobutanecarboxylate d'éthyle

On procède comme dans l'exemple 55 en utilisant comme réactif l'acide 2-(1*H*-1,2,3,4-tétraazol-1-yl)acétique.
*Point de fusion : 123-124°C*

| *Microanalyse élémentaire* : | | | | |
|---|---|---|---|---|
| | *C* | *H* | *N* | *Cl* |
| *% calculé* | *56,95* | *5,23* | *18,97* | *8,00* |
| *% trouvé* | *57,22* | *5,36* | *17,86* | *7,26* |

### EXEMPLE 72 : 1-{6-Chloro-2-[(1-méthyl-1H-imidazol-4-yl)carbonyl]-2,3,4,9-tétra hydro-1H-β-carbolin-1-yl}cyclobutanecarboxylate d'éthyle

On procède comme dans l'exemple 55 en utilisant comme réactif l'acide 1-méthyl-1*H*-imidazol-4-carboxylique.
*Point de fusion : 180-181°C*

| *Microanalyse élémentaire* : | | | | |
|---|---|---|---|---|
| | *C* | *H* | *N* | *Cl* |
| *% calculé* | *62,65* | *5,71* | *12,71* | *8,04* |
| *% trouvé* | *63,02* | *5,67* | *12,61* | *8,02* |

### EXEMPLE 73 : (1R)-1-[6-Chloro-2-(1H-imidazol-5-ylcarbonyl)-2,3,4,9-tétrahydro-1H-β-carbolin-1-yl]cyclobutanecarboxylate d'éthyle

Le composé de l'exemple 55 est élué sur une colonne chirale (CHIRALPAK AD) avec un éluant constitué d'éthanol, permettant d'isoler le produit attendu avec un excès énantiomérique de 98 %.
*Point de fusion : 140-142°C*

| *Microanalyse élémentaire* : | | | | |
|---|---|---|---|---|
| | *C* | *H* | *N* | *Cl* |
| *% calculé* | *61,90* | *5,43* | *13,12* | *8,30* |
| *% trouvé* | *61,71* | *5,52* | *12,82* | *9,02* |

### EXEMPLE 74 : (1S)-1-[6-Chloro-2-(1H-imidazol-5-ylcarbonyl)-2,3,4,9-tétrahydro-1H-β-carbolin-1-yl]cyclobutanecarboxylate d'éthyle

Le second composé élué lors de la chromatographie réalisée à l'exemple 73 correspond au produit attendu, avec un excès énantiomérique de 98 %.
*Point de fusion : 140-142°C*

| *Microanalyse élémentaire* : | | | | |
|---|---|---|---|---|
| | C | H | N | Cl |
| *% calculé* | *61,90* | *5,43* | *13,12* | *8,30* |
| *% trouvé* | *61,43* | *5,58* | *12,79* | *8,35* |

### EXEMPLE 75 : 1-{6-Chloro-2-[(1-méthyl-1H-imidazol-5-yl)carbonyl]-2,3,4,9-tétra hydro-1H-β-carbolin-1-yl}cyclobutanecarboxylate d'éthyle

On procède comme dans l'exemple 55 en utilisant comme réactif l'acide 1-méthyl-1*H*-imidazol-5-carboxylique.
*Point de fusion : 203-204°C*

| *Microanalyse élémentaire* : | | | | |
|---|---|---|---|---|
| | *C* | *H* | *N* | *Cl* |
| *% calculé* | *62,65* | *5,71* | *12,71* | *8,04* |
| *% trouvé* | *62,71* | *5,74* | *12,73* | *8,19* |

### EXEMPLE 76 : 1-{6-Chloro-2-[(5-méthyl-1H-imidazol-4-yl)carbonyl]-2,3,4,9-tétra hydro-1H-β-carbolin-1-yl}cyclobutanecarboxylate d'éthyle

On procède comme dans l'exemple 55 en utilisant comme réactif l'acide 5-méthyl-1*H*-imidazol-4-carboxylique.
*Point de fusion : 161-162°C*

| *Microanalyse élémentaire* : | | | | |
|---|---|---|---|---|
| | *C* | *H* | *N* | *Cl* |
| *% calculé* | *62,65* | *5,71* | *12,71* | *8,04* |
| *% trouvé* | *63,18* | *6,21* | *11,93* | *7,52* |

### EXEMPLE 77 : 1-{6-Chloro-2-[(4-méthyl-1,2,3-thiadiazol-5-yl)carbonylj-2,3,4,9-tétra hydro-1H-β-carbolin-1-yl}cyclobutanecarboxylate d'éthyle

On procède comme dans l'exemple 55 en utilisant comme réactif l'acide 4-méthyl-1,2,3-thiadiazol-5-carboxylique.
*Point de fusion : 162-163°C*

| *Microanalyse élémentaire* : | | | | | |
|---|---|---|---|---|---|
| | *C* | *H* | *N* | *Cl* | *S* |
| *% calculé* | *57,57* | *5,05* | *12,21* | *7,72* | *6,99* |
| *% trouvé* | *57,49* | *5,05* | *12,05* | *8,04* | *6,93* |

### EXEMPLE 78 : 1-[6-Chloro-2-(1,2,3-thiadiazol-4-ylcarbonyl)-2,3,4,9-tétrahydro-1H-β-carbolin-1-yl]cyclobutanecarboxylate d'éthyle

On procède comme dans l'exemple 55 en utilisant comme réactif l'acide 1,2,3-thiadiazol-4-carboxylique.
*Point de fusion : 224-225°C*

| *Microanalyse élémentaire* : | | | | | |
|---|---|---|---|---|---|
| | *C* | *H* | *N* | *Cl* | *S* |
| *% calculé* | *56, 69* | *4,76* | *12,59* | *7,97* | *7,21* |
| *% trouvé* | *56,90* | *4,96* | *12,28* | *8,00* | *7,13* |

### EXEMPLE 79 : 1-(2-Acétyl-6-chloro-2,3,4,9-tétrahydro-1H-β-carbolin-1-yl) cyclobutanecarboxylate d'éthyle

On procède comme dans l'exemple 55 en utilisant comme réactif le chlorure d'acétyle. *Point de fusion : 218-219°C*

| *Microanalyse élémentaire* : | | | | |
|---|---|---|---|---|
| | *C* | *H* | *N* | *Cl* |
| *% calculé* | *64,08* | *6,18* | *7,47* | *9,46* |
| *% trouvé* | *64,06* | *6,30* | *7,34* | *9,45* |

### EXEMPLE 80 : 1-(2-Acétyl-6-méthoxy-2,3,4,9-tétrahydro-1H-β-carbolin-1-yl) cyclobutanecarboxylate d'éthyle

On procède comme dans l'exemple 55 en utilisant comme substrat le composé de l'exemple 7 et comme réactif celui utilisé dans l'exemple 79.
*Point de fusion : 180-182°C*

| *Microanalyse élémentaire* : | | | |
|---|---|---|---|
| | *C* | *H* | *N* |
| *% calculé* | *68,09* | *7,07* | *7,56* |
| *% trouvé* | *68,18* | *7,28* | *7,56* |

### EXEMPLE 81 : 1-[6-Méthoxy-2-(2-furoyl)-2,3,4,9-tétrahydro-1H-β-carbolin-1-yl] cyclobutanecarboxylate d'éthyle

On procède comme dans l'exemple 55 en utilisant comme substrat le composé de l'exemple 7 et comme réactif le chlorure de 2-furoyle.
*Point de fusion : 149-150°C*

| *Microanalyse élémentaire* : | | | |
|---|---|---|---|
| | *C* | *H* | *N* |
| *% calculé* | *68,23* | *6,20* | *6,63* |
| *% trouvé* | *68,21* | *6,35* | *6,66* |

### EXEMPLE 82 : 1-[6-Chloro-2-(2-furoyl)-2,3,4,9-tétrahydro-1H-β-carbolin-1-yl] cyclobutanecarboxylate d'éthyle

On procède comme dans l'exemple 55 en utilisant comme réactif le chlorure de 2-furoyle. *Point de fusion : 195-196°C*

| *Microanalyse élémentaire* : | | | | |
|---|---|---|---|---|
| | *C* | *H* | *N* | *Cl* |
| *% calculé* | *64,71* | *5,43* | *6,56* | *8,30* |
| *% trouvé* | *64,61* | *5,55* | *6,54* | *8,29* |

### EXEMPLE 83 : Acide 1-(2-acétyl-6-chloro-2,3,4,9-tétrahydro-1H-β-carbolin-1-yl) cyclobutanecarboxylique

On procède comme dans l'exemple 55 en utilisant comme substrat le composé de l'exemple 32 et comme réactif le chlorure d'acétyle.
*Point de fusion : > 250°C*

| *Microanalyse élémentaire* : | | | | |
|---|---|---|---|---|
| | *C* | *H* | *N* | *Cl* |
| *% calculé* | *62,34* | *5,52* | *8,08* | *10,22* |
| *% trouvé* | *62,12* | *5,69* | *8,01* | *10,25* |

### EXEMPLE 84 : Acide 1-(2-acétyl-2,3,4,9-tétrahydro-1H-β-carbolin-1-yl) cyclobutanecarboxylique

On procède comme dans l'exemple 55 en utilisant comme substrat le composé de l'exemple 35 et comme réactif le chlorure d'acétyle.
*Point de fusion : 244-246°C*

| *Microanalyse élémentaire* : | | | |
|---|---|---|---|
| | *C* | *H* | *N* |
| *% calculé* | *69,21* | *6,45* | *8,97* |
| *% trouvé* | *69,03* | *6,70* | *8,69* |

### EXEMPLE 85 : Chlorhydrate de 1-(6-chloro-2,3,4,9-tétrahydro-1H-β-carbolin-1-yl) cyclobutanecarboxamide

A une solution de 1 g du composé de l'exemple 1, dans 20 ml de dioxane, 20 ml d'eau, et 10 ml de soude 1M est additionnée, à 0°C, 0,8 g de pyrocarbonate de t-butyle. Après 4 heures de réaction, le milieu réactionnel est dilué par addition d'eau, acidifié par une solution d'acide citrique à 5 %. Après extraction à l'acétate d'éthyle, séchage des phases organiques, et concentration sous pression réduite, le résidu obtenu est dilué dans 50 ml de tétrahydrofurane et 0,5 ml de N-méthylmorpholine. Après refroidissement du milieu à -10°C, 0,3 ml de chloroformiate d'éthyle sont additionnés, puis 0,2 ml d'une solution d'ammoniaque à 28 %. Après 12 heures de réaction, le solvant est distillé. Le résidu est alors repris dans un mélange eau/dichlorométhane. Après décantation, séchage et concentration sous pression réduite, le résidu est traité par une solution d'éthanol et d'éther diéthylique chlorhydrique permettant d'obtenir le produit souhaité sous forme de chlorhydrate.
*Point de fusion : 229-230°C*

| *Microanalyse élémentaire* : | | | | |
|---|---|---|---|---|
| | C | H | N | Cl |
| *% calculé* | *56,48* | *5,63* | *12,35* | *20,84* |
| *% trouvé* | *56,30* | *5,65* | *12,07* | *20,81* |

### EXEMPLE 86 : 1-[1-(Aminocarbonyl)cyclobutyl]-6-chloro-1,3,4,9-tétrahydro-2H-β-carbolin-2-carboxylate de tert-butyle

Le produit est un co-produit obtenu lors de la synthèse du composé de l'exemple 85.
*Point de fusion : 229-230°C*

| *Microanalyse élémentaire :* | | | | |
|---|---|---|---|---|
| | *C* | *H* | *N* | *Cl* |
| *% calculé* | *62,45* | *6,49* | *10,40* | *8,78* |
| *% trouvé* | *62,53* | *6,53* | *10,12* | *8,80* |

### EXEMPLE 87 : 1-[1-(Aminocarbonyl)cyclobutyl]-6-chloro-1,3,4,9-tétrahydro-2H-β-carbolin-2-carboxylate d'éthyle

A une solution de 1 g du composé de l'exemple 32 dans 50 ml de tétrahydrofurane et 0,5 ml de N-méthylmorpholine, sont additionnés à -10°C, 0,3 ml de chloroformiate d'éthyle, puis 0,2 ml d'une solution d'ammoniaque à 28 %. Après 12 heures de réaction, le solvant est distillé ; le résidu est ensuite repris dans un mélange eau/dichlorométhane. Après décantation, séchage et concentration sous pression réduite, on isole le produit attendu.
*Point de fusion : 200-201 °C*

| *Microanalyse élémentaire* : | | | | |
|---|---|---|---|---|
| | *C* | *H* | *N* | *Cl* |
| *% calculé* | *60,72* | *5,90* | *11,18* | *9,43* |
| *% trouvé* | *60,49* | *6,00* | *10,82* | *9,29* |

### EXEMPLE 88 : 1-(6-Chloro-2-méthyl-2,3,4,9-tétrahydro-1H-β-carbolin-1-yl) cyclobutanecarboxamide

On procède comme dans l'exemple 87 en utilisant comme substrat le composé de l'exemple 33.
*Point de fusion : 129-130°C*

| *Microanalyse élémentaire :* | | | | |
|---|---|---|---|---|
| | *C* | *H* | *N* | *Cl* |
| *% calculé* | *64,25* | *6,34* | *13,22* | *11,16* |
| *% trouvé* | *64,03* | *6,43* | *12,47* | *11,09* |

### EXEMPLE 89 : Chlorhydrate de 1-(6-chloro-2-méthyl-2,3,4,9-tétrahydro-1H-β-carbolin-1-yl)cyclohexanecarboxamide

On procède comme dans l'exemple 87 en utilisant comme substrat le composé de l'exemple 45.
*Point de fusion : 175-176°C*

### EXEMPLE 90 : 1-(2-Méthyl-2,3,4,9-tétrahydro-1H-β-carbolin-1-yl) cyclobutanecarboxamide

On procède comme dans l'exemple 87 en utilisant comme substrat le composé de l'exemple 46 et comme réactif l'amino carbaldéhyde.
*Point de fusion : 145-146°C*

### EXEMPLE 91 : 1-(2-Formyl-6-chloro-2,3,4,9-tétrahydro-1H-β-carbolin-1-yl) cyclobutanecarboxamide

Une réaction entre le composé de l'exemple 1, et l'amino carbaldéhyde en présence de méthylate de sodium dans le diméthylformamide permet d'obtenir le produit désiré.
*Point de fusion : > 260°C*

| *Microanalyse élémentaire :* | | | | |
|---|---|---|---|---|
| | *C* | *H* | *N* | *Cl* |
| *% calculé* | *61,54* | *5,47* | *12,66* | *10,68* |
| *% trouvé* | *61,36* | *5,61* | *12,26* | *10,75* |

### EXEMPLE 92 : Dichlorhydrate de 1-(6-chloro-2,3,4,9-tétrahydro-1H-β-carbolin-1-yl)-N-[2-(diméthylamino)éthyl]cyclobutanecarboxamide

On procède comme dans l'exemple 85 en utilisant comme réactif la 2-(N,N-diméthylamino)-éthylamine à la place de l'ammoniaque.
*Point de fusion : 229-231°C*

| *Microanalyse élémentaire* : | | | | |
|---|---|---|---|---|
| | *C* | *H* | *N* | *Cl* |
| *% calculé* | *53,64* | *6,53* | *12,51* | *23,75* |
| *% trouvé* | *53,13* | *6,39* | *12,31* | *24,35* |

### EXEMPLE 93 : 6-Chloro-1-[1-({[2-(diméthylamino)éthyl]amino}carbonyl)cyclobutyl] -1,3,4,9-tétrahydro-2H-β-carboline-2-carboxylate de tert-butyle

Le produit est obtenu en tant que co-produit lors de la synthèse du composé de l'exemple 92.
*Point de fusion : 84-85 °C*

| Microanalyse *élémentaire* : | | | | |
|---|---|---|---|---|
| | C | H | N | Cl |
| *% calculé* | *63,21* | *7,43* | *11,79* | *7,46* |
| *% trouvé* | *62,72* | *7,18* | *11,63* | *7,52* |

### EXEMPLE 94 : Acide 1-[2-(tert-butoxycarbonyl)-6-méthyl-2,3,4,9-tétrahydro-1H-β-carbolin-1-yl]cyclobutanecarboxylique

A une solution de 6,5 g du composé obtenu dans l'exemple 37, dans 50 ml de dioxane et 25 ml de soude 1M, sont additionnés 5,52 g de pyrocarbonate de *tert*-butyle. Après 20 heures de réaction à température ambiante, 50 ml d'acétate d'éthyle et 100 ml d'eau sont additionnés, puis le mélange réactionnel est acidifié jusqu'à pH = 2,3 par une solution de KHSO₄. Le précipité formé est filtré, rincé à l'eau et séché permettant d'isoler le produit attendu.
*Point de fusion : 217-218°C*

| *Microanalyse élémentaire* : | | | |
|---|---|---|---|
| | *C* | *H* | *N* |
| *% calculé* | *68,73* | *7,34* | *7,29* |
| *% trouvé* | *68,42* | *7,28* | *7,18* |

### EXEMPLE 95 : N-[2(Diméthylamino)éthyl)-1-(6-méthoxy-2-méthyl-2,3,4,9-tétra hydro-1H-β-carbolin-1-yl)cyclobutanecarboxamide

Le produit est obtenu par traitement thermique du composé de l'exemple 47 en présence d'un large excès de N,N-(diméthylamino)éthylamine.
*Point de fusion : 187-188°C*

| *Microanalyse élémentaire* : | | | |
|---|---|---|---|
| | *C* | *H* | *N* |
| *% calculé* | *68,72* | *8,39* | *14,57* |
| *% trouvé* | *68,48* | *8,31* | *14,52* |

### EXEMPLE 96 : 1-{6-Chloro-2-[(3-pyridinylamino)carbonyl]-2,3,4,9-tétrahydro-1H-β-carbolin-1-yl}cyclobutanecarboxylate d'éthyle

0,9 g de 3-pyridylcarbonylazide dans 60 ml de toluène sont chauffés au reflux pendant 1 h 30 sous atmosphère inerte. Après retour à température ambiante, 1,65 g du composé obtenu dans l'exemple 1 dilués dans 60 ml de dichlorométhane, sont additionnés. Le mélange réactionnel est agité 20 heures à température ambiante, puis le précipité formé est filtré, rincé puis séché permettant d'isoler le produit attendu.
*Point de fusion : 126-128°C*

### EXEMPLE 97 : 1-(1-{[(Benzyloxy)carbonyl]amino}cyclobutyl)-6-méthyl-1,3,4,9-tétrahydro-2H-β-carboline carboxylate de tert-butyle

2,8 g du composé obtenu dans l'exemple 94, dans 3,3 ml de diphénylphosphorylazide, 2,1 ml de triéthylamine et 5,2 ml d'alcool benzylique sont portés à reflux. Après traitement, une chromatographie sur gel de silice (dichlorométhane/méthanol) permet d'isoler le produit attendu.
*Point de fusion : 219-220°C*

| *Microanalyse élémentaire* : | | | |
|---|---|---|---|
| | *C* | *H* | *N* |
| *% calculé* | *71,14* | *7,20* | *8,58* |
| *% trouvé* | *70,38* | *7,17* | *8,16* |

### EXEMPLE 98 : Chlorhydrate de 1-(6-méthyl-2,3,4,9-tétrahydro-1H-β-carbolin-1-yl) cyclobutanamine

### Stade A1 : Phényl 1-(6-méthyl-2,3,4,9-tétrahydro-1H-β-carbolin-1-yl) cyclobutylcarbamate

0,9 g du composé obtenu dans l'exemple 97 sont solubilisés dans 50 ml d'acétate d'éthyle, dans lequel est fait buller de l'acide chlorhydrique gazeux. Après une heure de réaction, le précipité formé est filtré, rincé par de l'éther diéthylique et séché permettant d'isoler le produit attendu.
*Point de fusion : 244-245°C*

### Stade B1 : Chlorhydrate de 1-(6-méthyl-2,3,4,9-tétrahydro-1H-β-carbolin-1-yl) cyclobutanamine

0,5 g du composé obtenu dans le stade A1 sont hydrogénés par action d'hydrogène en présence de Palladium sur charbon à 10 % dans 50 ml d'éthanol. Après filtration et concentration sous pression réduite, le résidu est cristallisé dans l'éther diéthylique permettant d'isoler le produit attendu.
*Point de fusion : 180°C*

### EXEMPLE 99 : Chlorhydrate de 1-(6-tert-butyl-2,3,4,9-tétrahydro-1H-β-carbolin-1-yl)cyclobutanamine

On procède respectivement comme dans l'exemple 94, dans l'exemple 97 puis 98 des stades A1 à B1, en utilisant comme substrat le composé de l'exemple 39.
*Point de fusion : 245°C*

| *Microanalyse élémentaire* : | | | | |
|---|---|---|---|---|
| | *C* | *H* | *N* | *Cl* |
| *% calculé* | *66,35* | *8,45* | *12,58* | *10,62* |
| *% trouvé* | *66,80* | *8,37* | *11,96* | *9,91* |

### EXEMPLE 100 : 1-(6-Chloro-4,9-dihydro-3H-β-carbolin-1-yl)cyclobutanecarboxylate d'éthyle

Le produit est obtenu selon le protocole décrit dans l'exemple 1, sans la dernière étape de réduction par action du borohydrure de sodium.
*Point de fusion : 141-142°C*

| *Microanalyse élémentaire* : | | | | |
|---|---|---|---|---|
| | *C* | *H* | *N* | *Cl* |
| *% calculé* | *65,35* | *5,79* | *8,47* | *10,72* |
| *% trouvé* | *65,37* | *5,76* | *8,44* | *10,94* |

### EXEMPLE 101:1-(6-Bromo-4,9-dihydro-3H-β-carbolin-1-yl)cyclobutanecarboxylate d'éthyle

On procède comme dans l'exemple 100 en utilisant comme substrat celui utilisé dans l'exemple 2.
*Point de fusion : 132-133°C*

| *Microanalyse élémentaire* : | | | | |
|---|---|---|---|---|
| | *C* | *H* | *N* | *Br* |
| *% calculé* | *57,61* | *5,10* | *7,46* | *21,29* |
| *% trouvé* | *57,64* | *5,22* | *7,45* | *21,05* |

### EXEMPLE 102 : 1-(4,9-Dihydro-3H-β-carbolin-1-yl)cyclobutanecarboxylate d'éthyle

On procède comme dans l'exemple 100 en utilisant comme substrat celui utilisé dans l'exemple 8.
*Point de fusion : 121-122°C*

| *Microanalyse élémentaire* : | | | |
|---|---|---|---|
| | *C* | *H* | *N* |
| *% calculé* | *72,95* | *6,80* | *9,45* |
| *% trouvé* | *72,88* | *6,93* | *9,48* |

### EXEMPLE 103 : 1-(6-Méthyl-4,9-dihydro-3H-β-carbolin-1-yl)cyclobutane carboxylate d'éthyle

On procède comme dans l'exemple 100 en utilisant comme substrat celui utilisé dans l'exemple 5.
*Point de fusion : 101-103°C*

| *Microanalyse élémentaire* : | | | |
|---|---|---|---|
| | *C* | *H* | *N* |
| *% calculé* | *73,52* | *7,14* | *9,03* |
| *% trouvé* | *73,56* | *7,23* | *8,93* |

### EXEMPLE 104 : 1-(6-Méthoxy-4,9-dihydro-3H-β-carbolin-1-yl)cyclobutane carboxylate d'éthyle

On procède comme dans l'exemple 100 en utilisant comme substrat celui utilisé dans l'exemple 7.
*Point de fusion : 229-230°C*

| *Microanalyse élémentaire* : | | | |
|---|---|---|---|
| | *C* | *H* | *N* |
| *% calculé* | *69,92* | *6, 79* | *8,58* |
| *% trouvé* | *69,51* | *6,63* | *8,33* |

### EXEMPLE 105 : 1-(6-Chloro-9-éthyl-4,9-dihydro-3H-β-carbolin-1-yl) cyclobutanecarboxylate d'éthyle

On procède comme dans l'exemple 100 en utilisant comme substrat celui utilisé dans l'exemple 21.
*Point de fusion : 115-116°C*

| *Microanalyse élémentaire* : | | | | |
|---|---|---|---|---|
| | *C* | *H* | *N* | *Cl* |
| *% calculé* | *66,94* | *6,46* | *7,81* | *9,88* |
| *% trouvé* | *67, 82* | *6,62* | *7,85* | *9,94* |

### EXEMPLE 106 : 1-(6-Méthoxy-9H-β-carbolin-1-yl)cyclobutanecarboxylate d'éthyle

1,5 g du composé de l'exemple 104 sont mis au reflux dans 10 ml de xylène en présence de 0,25 g de Palladium sur charbon à 10 % pendant 96 heures. Après filtration et concentration sous pression réduite, une chromatographie sur gel de silice (dichlorométhane/acétate d'éthyle : 80/20) permet d'isoler le produit attendu.
*Point de fusion : 128-129°C*

| *Microanalyse élémentaire* : | | | |
|---|---|---|---|
| | *C* | *H* | *N* |
| *% calculé* | *70,35* | *6,21* | *8,64* |
| *% trouvé* | *70,36* | *6,22* | *8,63* |

### EXEMPLE 107 : 1-(9H-β-Carbolin-1-yl)cyclobutanecarboxylate d'éthyle

On procède comme dans l'exemple 106 en utilisant comme substrat le composé de l'exemple 102.
*Point de fusion : 144-145°C*

| *Microanalyse élémentaire* : | | | |
|---|---|---|---|
| | *C* | *H* | *N* |
| *% calculé* | *73,45* | *6,16* | *9,52* |
| *% trouvé* | *73,51* | *6,22* | *9,20* |

### EXEMPLE 108 : 1-(6-Chloro-9-éthyl-9H-β-carbolin-1-yl)cyclobutanecarboxylate d'éthyle

On procède comme dans l'exemple 106 en utilisant comme substrat le composé de l'exemple 105.
*Point de fusion : 131-132°C*

| *Microanalyse élémentaire* : | | | | |
|---|---|---|---|---|
| | *C* | *H* | *N* | *Cl* |
| *% calculé* | *67,32* | *5,93* | *7,85* | *9,93* |
| *% trouvé* | *67,19* | *6,08* | *7,57* | *10,12* |

### EXEMPLE 109 : Acide 1-(9H-β-carbolin-1-yl)cyclobutanecarboxylique

On procède comme dans l'exemple 32 en utilisant comme substrat le composé de l'exemple 107.
*Point de fusion : 115-116°C*

| *Microanalyse élémentaire* : | | | |
|---|---|---|---|
| | *C* | *H* | *N* |
| *% calculé* | *72,17* | *5,30* | *10,52* |
| *% trouvé* | *71,58* | *5,36* | *10,38* |

### EXEMPLE 110 : Dichlorhydrate de 1-(9H-β-carbolin-1-yl)-N-[2-(diméthylamino) éthyl]cyclobutanecarboxamide

Le produit est obtenu par traitement thermique du composé de l'exemple 107, en présence d'un large excès de N,N-(diméthylamino)éthylamine.
*Point de fusion* : *lyophilisat*

### EXEMPLE 111 : 1-(6-Méthoxy-9H-β-carbolin-1-yl)-N-[2-(diméthylamino)éthyl] cyclobutanecarboxamide

On procède comme dans l'exemple 110 en utilisant comme substrat le composé de l'exemple 106.
*Point de fusion : lyophilisat*

### EXEMPLE 112 : 1-(6-Hydroxy-9H-β-carbolin-1-yl)-N-[2-(diméthylamino)éthyl] cyclobutanecarboxamide

Le composé de l'exemple 111 est traité selon les conditions décrites dans l'exemple 15.
*Point de fusion : 182-183 °C*

### EXEMPLE 113 : Chlorhydrate de 1-[6-(trifluorométhyl)-2,3,4,9-tétrahydro-1H-β-carbolin-1-yl]cyclobutanecarboxylate d' éthyle

### Stade A : 2-[4-(Triéthylsilyl)-3-butynyl]-1H-isoindole-1,3(2H)-dione

108 g de tosylate de (4-triéthylsilanyl-but-3-yn-1-ol) dans 50 ml de diméthylformamide sont additionnés à une suspension de 72,3 g de Phtalimidate de potassium dans 450 ml de diméthylformamide. Après 4 heures à 60°C, le diméthylformamide est distillé. Le résidu est repris par un mélange 1/1 dichlorométhane/eau, extrait, séché, filtré puis concentré sous pression réduite permettant d'obtenir le produit attendu.

### Stade B : 2-{2-[2-(Triéthylsilyl)-5-(trifluorométhyl)-1H-indol-3-yl]éthyl}-1H-isoindole-1,3(2H)-dione

5,16 g de 2-iodo-4-trifluorométhylaniline, 11,6 g de composé obtenu au stade A, 0,75 g de dichloropalladium-diphénylphosphine ferrocène, 0,76 g de chlorure de lithium, et 3,81 g de bicarbonate de sodium dans 200 ml de diméthylformamide sont chauffés à 100°C sous atmosphère inerte. Après 30 heures, le solvant est distillé. Le résidu est repris par un mélange eau/dichlorométhane, extrait, séché, filtré puis concentré sous pression réduite. Une chromatographie sur gel de silice (dichlorométhane) permet d'isoler le produit attendu qui cristallise dans l'heptane.
*Point de fusion : < 25°C*

### Stade C : 2-[2-(Triéthylsilyl)-5-(trifluorométhyl)-1H-indol-3-yl]éthylamine

Un mélange de 6,25 g du composé obtenu au stade B et 3,5 ml d'hydrazine dans 150 ml d'éthanol est porté 1 heure à reflux, puis dilué par 20 ml d'HCl 4N et de nouveau porté à reflux 1 heure. Après dilution par 100 ml d'eau, l'éthanol est éliminé sous vide. Le résidu est alcalinisé par addition de soude, extrait au dichlorométhane. Les fractions organiques réunies et séchées sont concentrées sous pression réduite permettant d'isoler le produit attendu.
*Point de fusion : 56°C*

### Stade D : 2-[5-(Trifluorométhyl)-1H-indol-3-yl]éthylamine

3,8 g du composé obtenu au stade C sont agités 72 heures à température ambiante dans 200 ml de tétrahydrofurane, 10 ml d'une solution 1M de fluorure de tétrabutylammonium dans le tétrahydrofurane en présence d'1 g de tamis moléculaire 3Å. Après filtration de la résine, évaporation à sec, le résidu est repris dans 100 ml de HCl 1N et lavé par du dichlorométhane. La phase aqueuse est ensuite alcalinisée, extraite au dichlorométhane et les phases organiques réunies sont traitées de façon usuelle. Le produit obtenu cristallise sous forme de chlorhydrate dans un mélange éthanol/éther diéthylique chlorhydrique.
*Point de fusion : > 250°C*

### Stade E : Chlorhydrate de 1-[6-(trifluorométhyl)-2,3,4,9-tétrahydro-1H-β-carbolin-1-yl] cyclobutanecarboxylate d'éthyle

On procède comme dans l'exemple 1 en utilisant comme substrat le produit obtenu au stade D.
*Point de fusion : 237°C*

| *Microanalyse élémentaire* : | | | | |
|---|---|---|---|---|
| | *C* | *H* | *N* | *Cl* |
| *% calculé* | *56,65* | *5,50* | *6,95* | *8,80* |
| *% trouvé* | *56,62* | *5,60* | *6,92* | *8,90* |

### EXEMPLE 114 : Chlorhydrate de [1-(6-chloro-2,3,4,9-tétrahydro-1H-β-carbolin-1-yl)cyclobutyl]méthanol

10 g du composé de l'exemple 1 sont dissous dans 400 ml de tétrahydrofurane et 2 g de LiAlH₄ sont additionnés, en 30 minutes, sous atmosphère d'argon. Après 3 heures de réaction et hydrolyse du milieu réactionnel, les sels minéraux sont essorés et le filtrat est évaporé à sec. 7,9 g de produit sont obtenus qui sont ensuite transformés en chlorhydrate dans un mélange éthanol/éther diéthylique chlorhydrique pour conduire au produit attendu.

### EXEMPLE 115 : 1-(6-Chloro-2,3,4,9-tétrahydro-1H-β-carbolin-1-yl)cyclobutane carbonitrile

### Stade A : 1-(6-Chloro-2,3,4,9-tétrahydro-1H-β-carbolin-1-yl)cyclobutanecarbaldehyde

Un mélange de 2,8 g du composé de l'exemple 2 et 3,6 g du réactif de Dess-Martin dans 100 ml de dichlorométhane est agité 1 heure à 0°C. Après retour à température ambiante et évaporation du solvant, une chromatographie sur gel de silice (dichlorométhane) permet d'isoler le produit attendu.

### Stade B : 1-(6-Chloro-2,3,4,9-tétrahydro-1H-β-carbolin-1-yl)-N-hydroxycyclobutane carboxamide

Une solution de 1,2 g du composé obtenu au stade A dans 120 ml d'éthanol, 0,54 ml de triéthylamine et 0,27 g de chlorhydrate d'hydroxylamine est agitée pendant 6 heures puis concentrée sous pression réduite. Une chromatographie sur gel de silice (dichlorométhane/acétate d'éthyle : 98/2) permet d'isoler le produit attendu.

### Stade C: 1-(6-chloro-2,3,4,9-tétrahydro-1H-β-carbolin-1-yl)cyclobutanecarbonitrile

0,9 g du composé obtenu au stade B est agité pendant 2 heures, à température ambiante, dans une solution de 10 ml de dioxane, 0,8 ml de pyridine et 0,42 ml d'anhydride trifluoroacétique. Après évaporation du solvant, une chromatographie sur gel de silice du résidu (dichlorométhane/acétate d'éthyle : 96/4) permet d'isoler le produit attendu qui cristallise dans l'heptane.
*Point de fusion : 218-219°C*

| *Microanalyse élémentaire* : | | | | |
|---|---|---|---|---|
| | *C* | *H* | *N* | *Cl* |
| *% calculé* | *67,25* | *5,64* | *14,70* | *12,41* |
| *% trouvé* | *67,01* | *5,80* | *14,56* | *12,50* |

### EXEMPLE 116 : Chlorhydrate de 1-[1-(6-chloro-2,3,4,9-tétrahydro-1H-β-carbolin-1-yl)cyclobutyl)-1-propanone

### Stade A : 1-[1-(6-chloro-2,3,4,9-tétrahydro-1H-β-carbolin-1-yl)cyclobutyl]-1-propanol

2,32 g du composé obtenu au stade A de l'exemple 115 dans 150 ml de tétrahydrofurane sont agités, sous atmosphère inerte, pendant 4 heures à -30°C en présence de 15 ml de bromure d'éthyle magnésium en solution 1M dans le tétrahydrofurane. Après retour à température ambiante, le milieu est traité de façon classique, puis concentré sous pression réduite permettant d'isoler le produit attendu.

### Stade B : 1-[1-(6-chloro-2,3,4,9-tétrahydro-1H-β-carbolin-1-yl)cyclobutyl]-1-propanone

1 g du composé obtenu au stade A est traité selon le protocole du stade A de l'exemple 115 permettant d'obtenir le produit attendu.
*Point de fusion : 168-170°C (décomposition)*

| *Microanalyse élémentaire* : | | | | |
|---|---|---|---|---|
| | *C* | *H* | *N* | *Cl* |
| *% calculé* | *61,20* | *6,28* | *7,93* | *20,07* |
| *% trouvé* | *61,31* | *6,44* | *7,65* | *20,02* |

### ETUDE PHARMACOLOGIQUE DES COMPOSES DE L'INVENTION

### EXEMPLE 117 : Test des érections péniennes provoquées par le Ro 60-0175 (1,25 mg/kg, s.c.) chez le rat

Ce test (Eur. J. Pharma., 1997, 325, 9-12) permet d'évaluer la capacité d'agents pharmacologiques à inhiber les érections péniennes provoquées par l'administration de Ro 60-0175, un agoniste sélectif 5-HT_{2C}. Une inhibition est donc prédictive d'une activité antagoniste au niveau des récepteurs 5-HT_{2C}. Des rats mâles de souche Wistar (Iffa-Credo, France), pesant 120-140 g le jour de l'expérience, sont placées individuellement dans des boîtes d'observation (7,5 x 18 x 30 cm) en plexiglass, juste après avoir reçu le produit ou son véhicule. Trente minutes plus tard, les animaux reçoivent le Ro 60-0175 (1,25 mg/kg, s.c.) et le nombre d'érections effectuées lors des 30 minutes suivantes est comptabilisé. Lors de ce test, les composés de l'invention présentent respectivement pour les produits des exemples 1, 6, 7 et 18 des ID₅₀ (dose inhibitrice₅₀ exprimées en mg/kg, s.c.) de 0,9, 0,9, 1,1 et 0,5. Les composés de la présente invention possèdent donc une activité puissante dans ce domaine.

### EXEMPLE 118 : Activité antagoniste 5HT_{2B}

Les membranes sont préparées à partir de cellules CHO-5-HT_{2B} qui expriment le récepteur humain de la sérotonine 5HT_{2B}. Elles sont placées dans un tampon (Tris-HCl 50mM;pH = 7,4;CaCl₂ 4 mM) et placées à -80°C jusqu'à utilisation. Pour les expériences de binding 400 µl d'une suspension de membranes (concentration finale 50 µl prot/ml) sont incubés avec 50 µl de [³H]-LSD (concentration finale 1 nM) et 50 µl du composé à tester, pendant 1 heure à 37°C, puis filtrés sur des filtres pré-incubés dans 0,1 % de polyéthylèneimine. Lors de ce test, les composés de l'invention se comportent bien comme des antagonistes 5HT_{2B} et présentent des IC₅₀ de l'ordre nanomolaire.

### EXEMPLE 119 : Composition pharmaceutique : comprimés

| Formule de préparation pour 1000 comprimés dosés à 5 mg | |
|---|---|
| Composé de l'exemple 1 | 5 g |
| Hydroxypropylcellulose | 2 g |
| Amidon de blé | 10 g |
| Lactose | 100 g |
| Stéarate de magnésium | 2 g |
| Talc | 2g |

## Revendications

1. Composés de formule (I): 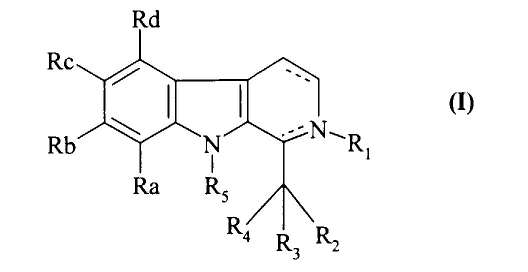 dans laquelle :
---- représente une liaison simple ou double pouvant éventuellement conférée un caractère aromatique au cycle qui les porte,
R₁ représente l'un des groupements choisis parmi :
◆ hydrogène,
◆ alkyle (C₁-C₆) linéaire ou ramifié,
◆ -R₆-aryle, -R₆-cycloalkyle, -R₆-hétérocycle, groupements dans lesquels R₆ représente un groupement alkylène (C₁-C₆) linéaire ou ramifié,
◆ -CO₂R₇ dans lequel R₇ représente un groupement alkyle (C₁-C₆) linéaire ou ramifié, aryle, cycloalkyle, un hétérocycle, un groupement -R₆-aryle, -R₆-cycloalkyle ou -R₆-hétérocycle avec R₆ tel que défini précédemment,
◆ -COR₈ dans lequel R₈ représente un atome d'hydrogène, un groupement alkyle (C₁-C₆) linéaire ou ramifié, aryle, cycloalkyle, un hétérocycle, un groupement -R₆-aryle, -R₆-cycloalkyle ou -R₆-hétérocycle avec R₆ tel que défini précédemment,
◆ et -CONH-R₈ dans lequel R₈ est tel que défini précédemment,
ou R₁ n'existe pas lorsque l'atome d'azote qui le porte, porte déjà une double liaison intracyclique,
R₂ représente l'un des groupements choisis parmi :
◆ cyano,
◆ -CO₂R₈ dans lequel R₈ est tel que défini précédemment,
◆ -CONHR₈ dans lequel R₈ est tel que défini précédemment,
◆ monoalkyl(C₁-C₆)aminoalkyl(C₁-C₆)aminocarbonyle, dialkylamino(C₁-C₆)aminoalkyl-(C₁-C₆)aminocarbonyle, les parties alkyles de chacun de ces groupements pouvant être linéaires ou ramifiées,
◆ -NR₈R₉ dans lequel R₈ est tel que défini précédemment et R₉ représente un groupement tel que R₈ est défini,
◆ -NH-CO₂R₇ dans lequel R₇ est tel que défini précédemment,
◆ et -COR₈ dans lequel R₈ est tel que défini précédemment,
R₃ et R₄ forment ensemble un cycloalkyle (C₃-C₁₀),
R₅ représente un atome d'hydrogène, un groupement alkyle (C₁-C₆) linéaire ou ramifié, ou arylalkyle (C₁-C₆) linéaire ou ramifié,
Ra, Rb, Rc, Rd, identiques ou différents, indépendamment l'un de l'autre, représentent un groupement choisi parmi atome d'hydrogène, d'halogène, groupement alkyle (C₁-C₆) linéaire ou ramifié, hydroxy, alkoxy (C₁-C₆) linéaire ou ramifié, trihalogénoalkyle (C₁-C₆) linéaire ou ramifié, trihalogénoalkoxy (C₁-C₆) linéaire ou ramifié, nitro, cyano, amino, alkyle(C₁-C₆)amino linéaire ou ramifié, dialkyle(C₁-C₆)amino linéaire ou ramifié, aryle, arylalkyle (C₁-C₆) linéaire ou ramifié, carboxy, alkylcarbonyloxy (C₁-C₆) linéaire ou ramifié, acyle (C₁-C₆) linéaire ou ramifié, aryloxy, et arylalkoxy (C₁-C₆) linéaire ou ramifié,
leurs isomères ainsi que leurs sels d'addition à un acide ou à une base pharmaceutiquement acceptable,
étant entendu que :
- par cycloalkyle, on comprend un groupement mono ou bicyclique, saturé (ou comportant éventuellement une ou plusieurs insaturations, celles-ci ne conférant pas un caractère aromatique au système cyclique), comportant de 3 à 10 atomes de carbone, éventuellement substitué par un ou plusieurs groupements, identiques ou différents, choisis parmi halogène, hydroxy, alkyle (C₁-C₆) linéaire ou ramifié, et alkoxy (C₁-C₆) linéaire ou ramifié,
- par aryle, on comprend un groupement phényle, naphtyle, tétrahydronaphtyle, dihydronaphtyle, indényle ou indanyle, chacun de ces groupements étant éventuellement substitué de façon identique ou différente, par un ou plusieurs groupements choisis parmi halogène, groupements hydroxy, cyano, nitro, alkyle (C₁-C₆) linéaire ou ramifié, alkoxy (C₁-C₆) linéaire ou ramifié, amino, alkyle (C₁-C₆)amino linéaire ou ramifié, dialkyle(C₁-C₆)amino linéaire ou ramifié, aryloxy, arylalkoxy (C₁-C₆) linéaire ou ramifié, trihalogénoalkyle (C₁-C₆) linéaire ou ramifié, acyle (C₁-C₆) linéaire ou ramifié, alkoxycarbonyle (C₁-C₆) linéaire ou ramifié, alkylaminocarbonyle (C₁-C₆) linéaire ou ramifié, et oxo,
- par hétérocycle, on comprend un groupement mono ou bicyclique, saturé ou insaturé, à caractère aromatique ou non aromatique, de 5 à 12 chaînons, contenant un, deux ou trois hétéroatomes, identiques ou différents, choisis parmi oxygène, azote et soufre, étant entendu que l'hétérocycle peut être éventuellement substitué, de façon identique ou différente, par un ou plusieurs groupements choisis parmi halogène, hydroxy, alkyle (C₁-C₆) linéaire ou ramifié, alkoxy (C₁-C₆) linéaire ou ramifié, nitro, oxo, et amino (substitué éventuellement par un ou deux groupements alkyle (C₁-C₆) linéaire ou ramifié).

2. Composés de formule (I) selon la revendication 1 caractérisés en ce que R₃ et R₄ forment ensemble un cycloalkyle (C₃-C₁₀), monocyclique et saturé, éventuellement substitué par un ou plusieurs groupements, identiques ou différents, choisis parmi halogène, hydroxy, alkyle (C₁-C₆) linéaire ou ramifié, et alkoxy (C₁-C₆) linéaire ou ramifié, leurs isomères ainsi que leurs sels d'addition à un acide ou à une base pharmaceutiquement acceptable.

3. Composés de formule (I) selon l'une quelconque des revendications 1 ou 2 caractérisés en ce que R₃ et R₄ forment ensemble un cycloalkyle (C₄-C₆) monocyclique, saturé, et non substitué, leurs isomères ainsi que leurs sels d'addition à un acide ou à une base pharmaceutiquement acceptable.

4. Composés de formule (I) selon l'une quelconque des revendications 1 à 3 caractérisés en ce que R₃ et R₄ forment ensemble un cyclobutyle, leurs isomères ainsi que leurs sels d'addition à un acide ou à une base pharmaceutiquement acceptable.

5. Composés de formule (I) selon la revendication 1 caractérisés en ce que R₁ représente un atome d'hydrogène ou un groupement -COR₈ dans lequel R8 est tel que défini dans la formule (I), leurs isomères ainsi que leurs sels d'addition à un acide ou à une base pharmaceutiquement acceptable.

6. Composés de formule (I) selon l'une quelconque des revendications 1 ou 5 caractérisés en ce que R₁ représente un groupement -COR₈ₐ dans lequel R₈ₐ représente un groupement aryle ou un hétérocycle, leurs isomères ainsi que leurs sels d'addition à un acide ou à une base pharmaceutiquement acceptable.

7. Composés de formule (I) selon la revendication 1 caractérisés en ce que R₂ représente un groupement de formule -CO₂R₈ dans lequel R₈ est tel que défini dans la formule (I), leurs isomères ainsi que leurs sels d'addition à un acide ou à une base pharmaceutiquement acceptable.

8. Composés de formule (I) selon l'une quelconque des revendications 1 ou 7 caractérisés en ce que R₂ représente un groupement de formule -CO₂R_{8b} dans lequel R_{8b} représente un groupement alkyle (C₁-C₆) linéaire ou ramifié ou un cycloalkyle, leurs isomères ainsi que leurs sels d'addition à un acide ou à une base pharmaceutiquement acceptable.

9. Composés de formule (I) selon l'une quelconque des revendications 1 ou 8 caractérisés en ce que R₂ représente un groupement de formule -CO₂R_{8b} dans lequel R_{8b} représente un groupement éthyle ou cyclopentyle, leurs isomères ainsi que leurs sels d'addition à un acide ou à une base pharmaceutiquement acceptable.

10. Composés de formule (I) selon la revendication 1 caractérisés en ce que R₅ représente un atome d'hydrogène, leurs isomères ainsi que leurs sels d'addition à un acide ou à une base pharmaceutiquement acceptable.

11. Composés de formule (I) selon la revendication 1 caractérisé en ce qu'ils appartiennent à la formule (I'): 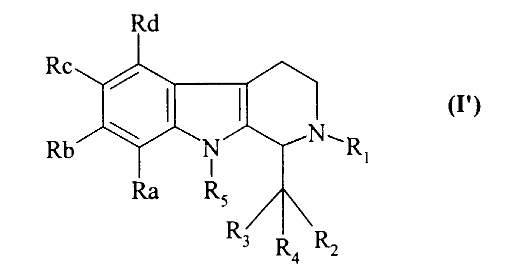 dans laquelle R₁, R₂, R₃, R₄, R₅, Ra, Rb, Rc et Rd sont tels que définis dans la formule (I), leurs isomères ainsi que leurs sels d'addition à un acide ou à une base pharmaceutiquement acceptable.

12. Composé de formule (I) selon la revendication 1 qui est le 1-(6-chloro-2,3,4,9-tétrahydro-1*H*-β-carbolin-1-yl)cyclobutanecarboxylate de cyclopentyle, ses isomères ainsi que ses sels d'addition à un acide ou à une base pharmaceutiquement acceptable.

13. Composé de formule (I) selon la revendication 1 qui est le 1-(6-bromo-2,3,4,9-tétrahydro-1*H*-β-carbolin-1-yl)cyclobutanecarboxylate d'éthyle, ses isomères ainsi que ses sels d'addition à un acide ou à une base pharmaceutiquement acceptable.

14. Composé de formule (I) selon la revendication 1 qui est le 1-[6-chloro-2-(1*H*-imidazol-5-ylcarbonyl)-2,3,4,9-tétrahydro-1*H*-β-carbolin-1-yl]cyclobutanecarboxylate d'éthyle, ses isomères ainsi que ses sels d'addition à un acide ou à une base pharmaceutiquement acceptable.

15. Composé de formule (I) selon la revendication 1 qui est le 1-(6-méthyl-2,3,4,9-tétrahydro-1*H*-β-carbolin-1-yl)cyclobutanecarboxylate d'éthyle, ses isomères ainsi que ses sels d'addition à un acide ou à une base pharmaceutiquement acceptable.

16. Composé de formule (I) selon la revendication 1 qui est le 1-(5,6-dichloro-2,3,4,9-tétrahydro-1*H*-β-carbolin-1-yl)cyclobutanecarboxylate d'éthyle, ses isomères ainsi que ses sels d'addition à un acide ou à une base pharmaceutiquement acceptable.

17. Composé de formule (I) selon la revendication 1 qui est le 1-(6-chloro-2,3,4,9-tétrahydro-1*H*-β-carbolin-1-yl)cyclobutanecarboxylate d'éthyle, ses isomères ainsi que ses sels d'addition à un acide ou à une base pharmaceutiquement acceptable.

18. Composé de formule (I) selon la revendication 1 qui est le 1-(6,7-dichloro-2,3,4,9-tétrahydro-1*H*-β-carbolin-1-yl)cyclobutanecarboxylate d'éthyle, ses isomères ainsi que ses sels d'addition à un acide ou à une base pharmaceutiquement acceptable.

19. Composé de formule (I) selon la revendication 1 qui est le 1-(6-méthoxy-2,3,4,9-tétrahydro-1*H*-β-carbolin-1-yl)cyclobutanecarboxylate d'éthyle, ses isomères ainsi que ses sels d'addition à un acide ou à une base pharmaceutiquement acceptable.

20. Procédé de préparation des composés de formule (I) caractérisé en ce qu'on utilise comme produit de départ un composé de formule (**II**) : 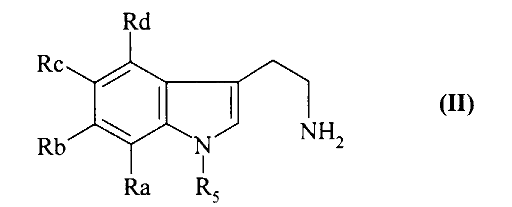 dans lequel Ra, Rb, Rc, Rd et R₅ ont la même signification que dans la formule (I),
composé de formule (II) qui est mis à réagir, selon des conditions de synthèse de type couplage peptidique, avec un composé de formule (**III**) : 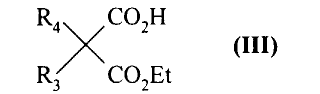 dans laquelle R₃ et R₄ sont tels que définis dans la formule (I),
pour conduire au composé de formule (**IV**) : 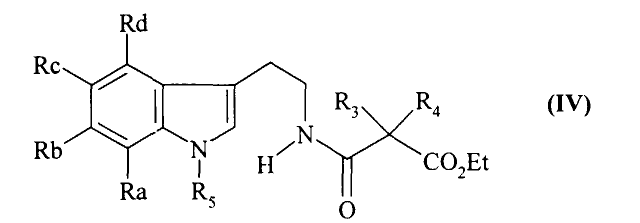 dans laquelle Ra, Rb, Rc, Rd, R₃, R₄ et R₅ sont tels que définis précédemment, composés de formule (IV) que l'on traite en présence d'oxychlorure de phosphore dans un solvant tel que le toluène ou le benzène, pour conduire aux composés de formule (**I/a**), cas particulier des composés de formule (I): 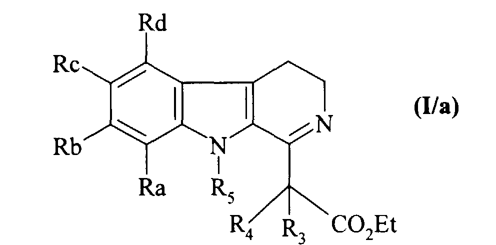 dans laquelle Ra, Rb, Rc, Rd, R₃, R₄ et R₅ sont tels que définis précédemment,
composés de formule (I/a) qui sont :
* soit réduits selon des conditions classiques de synthèse organique, pour conduire aux composés de formule (**I/b**), cas particulier des composés de formule (I): 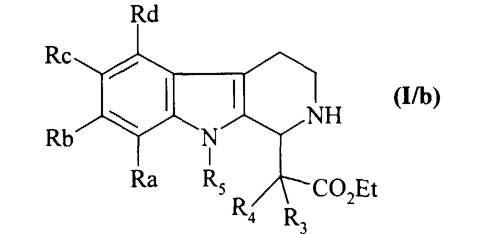 dans laquelle Ra, Rb, Rc, Rd, R₃, R₄ et R₅ sont tels que définis précédemment,
composés de formule (I/b) que l'on traite en condition basique en présence d'un composé de formule (**V**) :
R₁ - X (**V**)
dans laquelle R₁ est tel que défini dans la formule (I) et X représente un groupe partant usuellement utilisé en synthèse organique, pour conduire aux composés de formule (**I/c**), cas particulier des composés de formule (I): 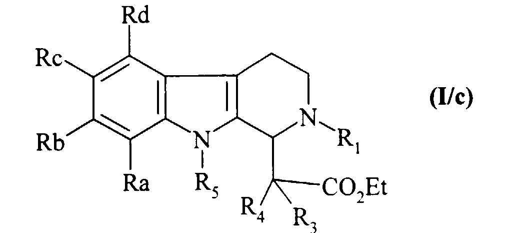
* soit soumis à des agents oxydants usuellement utilisés en synthèse organique pour conduire aux composés de formule (**I/d**), cas particulier des composés de formule (I): 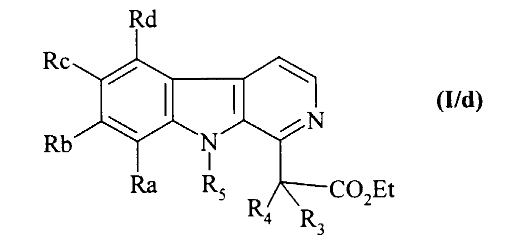 dans laquelle Ra, Rb, Rc, Rd, R₃, R₄ et R₅ sont tels que définis précédemment,
l'ensemble des composés de formule (I/a), (I/b), (I/c) et (I/d) forment les composés de formule (**I/e**), cas particulier des composés de formule (I): 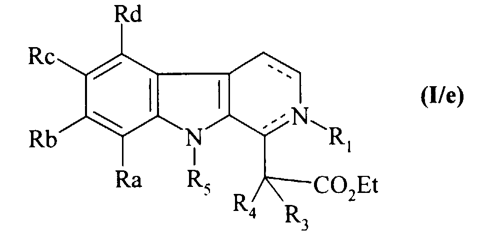 dans laquelle Ra, Rb, Rc, Rd, R₁, R₃, R₄ et R₅ ont la même définition que dans la formule (I), composé de formule (I/e),
* que l'on soumet à des conditions de transestérification en présence d'un acide de Lewis, et d'un composé de formule (**VI**) :
R₇ - OH (**VI**)
dans laquelle R₇ est tel que défini dans la formule (I),
pour conduire, aux composés de formule (**I/f**), cas particulier des composés de formule (I) : 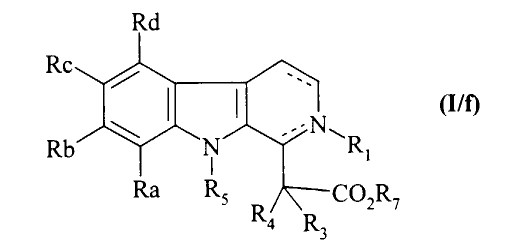 dans laquelle Ra, Rb, Rc, Rd, R₁, R₃, R₄, R₅ et R₇ sont tels que définis précédemment,
* ou que l'on hydrolyse en condition basique, pour conduire aux composés de formule (**I/g**), cas particulier des composés de formule (I): 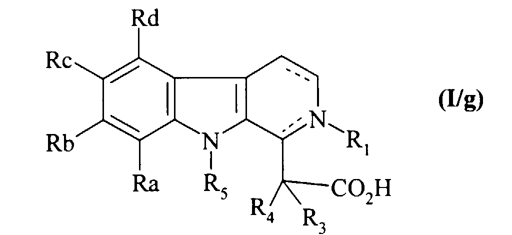 dans laquelle Ra, Rb, Rc, Rd, R₁, R₃, R₄ et R₅ sont tels que définis précédemment, composé de formule (**I/g**) que l'on traite :
◆ *soit,* selon des conditions classiques d'amidification, par un composé de formule (**VII**):
R₈ - NH₂ (**VII**)
dans laquelle R₈ est tel que défini dans la formule (I), pour conduire aux composés de formule (**I/h**), cas particulier des composés de formule (I): 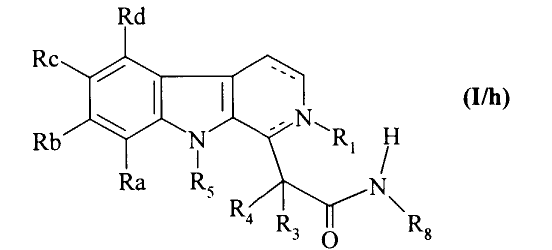 dans laquelle Ra, Rb, Rc, Rd, R₁, R₃, R₄, R₅ et R₈ sont tels que définis précédemment, composés de formule (I/h), dans le cas particulier où R₈ représente un atome d'hydrogène, dont la fonction amide primaire est transformée en fonction nitrile selon des conditions classiques de la synthèse organique, pour conduire aux composés de formule (**I/i**), cas particulier des composés de formule (I): 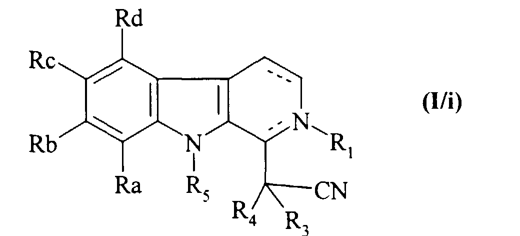 dans laquelle Ra, Rb, Rc, Rd, R₁, R₃, R₄ et R₅ sont tels que définis précédemment,
◆ *soit* dont on transforme la fonction acide carboxylique en aldéhyde, par une suite de réaction de réduction puis oxydation selon des conditions usuelles de la chimie organique, pour conduire aux composés de formule (**I/j**), cas particulier des composés de formule (I): 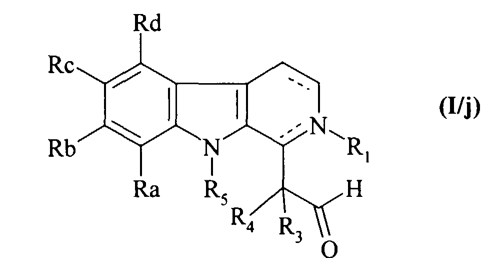 dans laquelle Ra, Rb, Rc, Rd, R₁, R₃, R₄ et R₅ sont tels que définis précédemment, composés de formule (I/j) qui est mis en présence d'un composé de formule (**VIII**):
R₇ - M - X **(VIII)**
dans laquelle R₇ est tel que défini dans la formule (I), M représente un atome de métal tel qu'un métal alcalin ou du magnésium et X représente un groupement partant tel qu'un atome d'halogène, pour conduire intermédiairement aux composés de formule (**IX**): 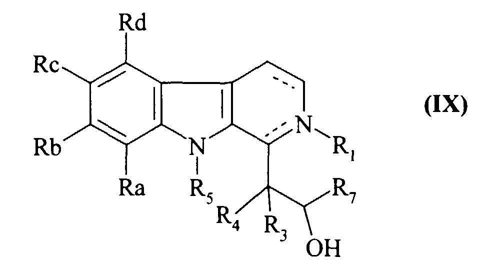 dans laquelle Ra, Rb, Rc, Rd, R₁, R₃, R₄, R₅ et R₇ sont tels que définis précédemment, composés de formule (IX) qui sont oxydés au moyen d'un oxydant couramment utilisé en synthèse organique,
pour conduire aux composés de formule (**I/k**), cas particulier des composés de formule (I): 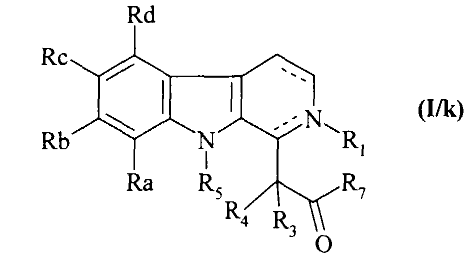 dans laquelle Ra, Rb, Rc, Rd, R₁, R₃, R₄, R₅ et R₇ sont tels que définis précédemment,
◆ *soit* que l'on traite par du diphénylphosphoryle azide, en présence de triéthylamine et d'un composé de formule (VI) R7-OH, tel que défini précédemment, pour conduire aux composés de formule (**I/l**), cas particulier des composés de formule (I): 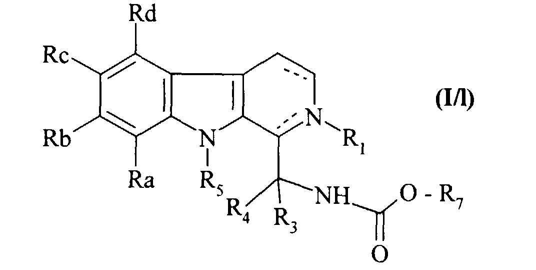 dans laquelle Ra, Rb, Rc, Rd, R₁, R₃, R₄, R₅ et R₇ sont tels que définis précédemment, composés de formule (I/1) dans le cas particulier où R₇ représente un groupement benzyle, que l'on soumet à des conditions d'hydrogénolyse en présence de palladium sur charbon, pour conduire aux composés de formule (**I/m**), cas particulier des composés de formule (I): 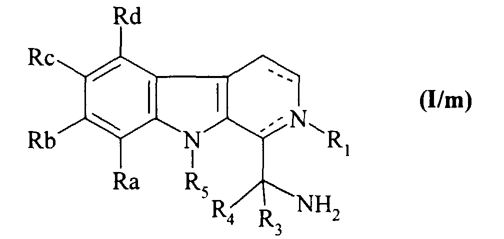 composés de formule (I/m) dont la fonction amine primaire est transformée en fonction amine secondaire ou tertiaire, selon les méthodes classiques, pour conduire aux composés de formule (**I/n**), cas particulier des composés de formule (I): 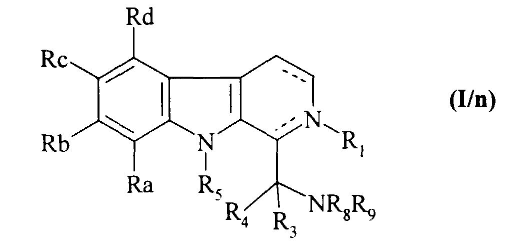 dans laquelle Ra, Rb, Rc, Rd, R₁, R₃, R₄, R₅, R₈ et R₉ ont la même définition que dans la formule (I), étant entendu que, dans ce cas, R₈ et R₉ ne représentent pas simultanément un atome d'hydrogène,
les composés (I/a) à (I/n) formant l'ensemble des composés de l'invention, que l'on purifie, le cas échéant, selon une technique classique de purification, qui peuvent, si on le désire, être séparés en leurs différents isomères, selon une technique classique de séparation, et que l'on transforme, le cas échéant, en leurs sels d'addition à un acide ou à une base pharmaceutiquement acceptable.

21. Compositions pharmaceutiques contenant comme principe actif au moins un composé de formule (I) selon l'une quelconque des revendications 1 à 17, seul ou en combinaison avec un ou plusieurs excipients ou véhicules inertes, non toxiques, pharmaceutiquement acceptables.

22. Compositions pharmaceutiques selon la revendication 19 contenant au moins un principe actif selon l'une quelconque des revendications 1 à 17, utiles pour le traitement de la dépression, de la psychose, de la schizophrénie, de la phobie, de l'anxiété, des attaques de panique, des troubles du sommeil, des troubles de l'appétit, des troubles impulsifs et agressifs, des troubles sexuels, et de la migraine.
